(19) **Europäisches Patentamt**
**European Patent Office**
**Office européen des brevets**

(11) **EP 4 763 828 A1**

(12) # EUROPEAN PATENT APPLICATION

(43) Date of publication:
24.06.2026 Bulletin 2026/26

(21) Application number: 25155326.9

(22) Date of filing: 31.01.2025

(51) International Patent Classification (IPC):
*C07C 29/151* $^{(2006.01)}$   *C07C 31/04* $^{(2006.01)}$

(52) Cooperative Patent Classification (CPC):
(C-Sets available)
**C07C 29/1518**                                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.12.2024 CY 2400003
19.12.2024 GR 20240100904**

(71) Applicant: **Pyletech Energy Limited
2007 Strovolos Nicosia (CY)**

(72) Inventor: **Livanos, Georgios
19009 Rafina (GR)**

(74) Representative: **Abel & Imray LLP
Westpoint Building
James Street West
Bath BA1 2DA (GB)**

(54) **METHODS AND SYSTEMS FOR CONVERTING BIOMASS TO METHANOL**

(57)     The present disclosure relates to a system (100) for converting biomass to methanol and methods of performing the same. The system (100) comprises a gasification reactor (102), air-separation unit (112), electrolysis unit (114), water-gas shift reactor (124) and methanol synthesis reactor (116). Preferably, the system (100) comprises a bypass conduit (136) for diverting a portion of syngas produced by the gasification reactor (102) so that it does not pass through the water-gas shift reactor (124).

Fig. 1

EP 4 763 828 A1

(52) Cooperative Patent Classification (CPC): (Cont.)

C-Sets
**C07C 29/1518, C07C 31/04**

**Description**

Field of the Invention

The present invention concerns systems and methods for converting biomass to methanol, such as green methanol. More particularly, but not exclusively, the invention concerns a system comprising a gasification reactor, air-separation unit, electrolysis unit, water-gas shift reactor, bypass conduit, bypass control system and methanol synthesis reactor and methods of converting biomass to methanol in said system. Advantageously, the bypass conduit directs syngas upstream of the water-gas shift reactor to downstream of the water-gas shift reactor without passing though the water-gas shift reactor. Advantageously, the bypass control system controls the flow of syngas through the bypass conduit.

Background of the Invention

Biomass-derived fuels (so-called 'biofuels') are an attractive alternative to traditional fossil fuels. Biofuels are derived from plant material so have a lower carbon footprint than fossil fuels and are also renewable. Waste agricultural or residual forestry biomass is particularly attractive as a biomass source.

Methanol derived from biomass is appealing as a fuel for the transportation industry, especially for high power demand vehicles such as aircraft, marine vessels and lorries, where battery storage cannot meet power and energy demand. Methanol can be blended with gasoline or used alone, with only minor modifications needed to existing fuel-delivery infrastructure and combustion engines.

An established route for converting biomass to methanol uses a two-stage process. In the first stage biomass is converted to syngas in a gasification process. In the second stage syngas is converted to methanol in a methanol synthesis reactor.

Syngas comprises a mixture of carbon dioxide, carbon monoxide and hydrogen ($CO$, $CO_2$ and $H_2$). The ratio of $CO$, $CO_2$ and $H_2$ can be varied by appropriate choice of gasification reactor and reaction conditions, but typically the syngas produced by gasification of biomass is rich in $CO$ and $H_2$ with $CO_2$ as a minor component. Syngas may be converted in a methanol synthesis reactor by hydrogenation of $CO$ and/or $CO_2$ according to Eq. 1 and Eq. 2.

$$2H_2 + CO \; 4 \; CH_3OH \qquad \text{(Eq. 1)}$$

$$3H_2 + CO_2 \; 4 \; CH_3OH + H_2O \qquad \text{(Eq. 2)}$$

The stoichiometric number (SN) of the syngas is determined by the concentration of $CO$, $CO_2$ and $H_2$ of the syngas, as shown in Eq. 3. Ideally, when methanol synthesis Eq. 1 represents the major reaction occurring in the methanol synthesis reactor, the stoichiometric number (SN) of the syngas feed will be in the region of 2.0 - 2.1. However, syngas produced from biomass in the gasification reactor is typically below stoichiometric in hydrogen content.

$$SN = ([H_2] - [CO_2])/([CO] + [CO_2]) \qquad \text{(Eq. 3)}$$

To increase the hydrogen content of the syngas, the syngas may undergo a water-gas shift process in a water-gas shift unit. The water-gas shift process is shown in Eq. 4. Reaction conditions and catalyst can be selected to promote the forward reaction, thus increasing the hydrogen content of the syngas. In addition to increasing the hydrogen content of the syngas, the carbon dioxide content is also increased. Carbon dioxide can be removed from the syngas, for example by an acid gas removal unit, and sequestered or released to the atmosphere as biogenic carbon dioxide.

$$CO + H_2O \leftrightarrow CO_2 + H_2 \qquad \text{(Eq. 4)}$$

US 2022/0220052 A1 discloses recycling unreacted hydrogen discharged from a methanol reactor back to the syngas feed to improve the stoichiometric number. However further methods of adjusting the stoichiometric number of syngas are needed.

A disadvantage of using the water-gas shift process is that a proportion of the carbon content of the biomass is lost as carbon dioxide. This limits the yield of methanol that can be formed. US 9,416,077 B2 discloses a system in which a water-gas shift reactor is excluded and hydrogen supplied by a steam methane reformer is added downstream of the gasification reactor to adjust the $H_2:CO$ ratio for methanol synthesis.

However, there remains a need to develop improved systems and methods of forming methanol from biomass.

The present invention seeks to mitigate the above-mentioned problems. Additionally or alternatively, the present invention seeks to provide an improved system and method for forming methanol from biomass with increased efficiency and flexibility.

Summary of the Invention

The present invention provides, according to a first aspect, a system for converting biomass to methanol, the system comprising:

a gasification reactor comprising a biomass feed inlet and a syngas outlet;
a water-gas shift reactor located downstream of the gasification reactor and comprising a syngas stream inlet and hydrogen-rich syngas stream outlet;
a first methanol synthesis reactor located downstream of the water-gas shift reactor;
optionally, a bypass conduit fluidly connecting the syngas outlet of the gasification reactor with the first methanol synthesis reactor, wherein the bypass conduit does not pass through the water-gas shift reactor;
optionally, a bypass control system for controlling the flow of syngas to the water-gas shift reactor and the flow of syngas through the bypass conduit;
an air-separation unit comprising an air inlet and an oxygen outlet; and,
an electrolysis unit comprising a water inlet, an oxygen outlet and a hydrogen outlet;
wherein the gasification reactor is arranged to receive:

(i) oxygen from the oxygen outlet of the air-separation unit; and/or,
(ii) oxygen from the oxygen outlet of the electrolysis unit;
and the first methanol synthesis reactor is arranged to receive:
(iii) hydrogen-rich syngas from the hydrogen-rich syngas stream outlet of water-gas shift reactor; and/or,
(iv) syngas from the bypass conduit and hydrogen from the hydrogen outlet of the electrolysis unit.

Optionally, the gasification reactor is arranged to receive:

(i) oxygen from the oxygen outlet of the air-separation unit; and,
(ii) oxygen from the oxygen outlet of the electrolysis unit;
and the first methanol synthesis reactor is arranged to receive:
(iii) hydrogen-rich syngas from the hydrogen-rich syngas stream outlet of water-gas shift reactor; and,
(iv) syngas from the bypass conduit and hydrogen from the hydrogen outlet of the electrolysis unit.

Optionally, the system has a capacity of at least 3000 tonnes of methanol per day, preferably at least 4000 tonnes of methanol per day.

In operation, the gasification reactor may receive oxygen from either or both of the air-separation unit and the electrolysis unit. Optionally, the air-separation unit and/or electrolysis unit are powered by green electricity. It will be understood that green electricity is electricity generated by renewable energy such as wind, solar, tidal or geothermal energy. Having a system with both an air-separation unit and an electrolysis unit allows flexibility in response to green energy supply and price.

As described herein, the air-separation unit consumes less power than the electrolysis unit. When electricity supply is reduced or expensive, the system may be operated in a 'gasification-only mode' in which the air-separation unit is in operation and the electrolysis unit is not in operation. Conversely, when energy supply and price makes it economically viable to do so, it may be desirable to operate the system in a 'full-carbon recovery mode' in which the electrolysis unit is in operation and the air-separation unit is not in operation. Operating the system in full-carbon recovery mode is particularly advantageous as the electrolysis unit also produces a hydrogen stream for use elsewhere in the system to improve conversion of carbon to methanol. The system may also be operated in an intermediate mode lying between the extremes of the gasification-only and full-carbon recovery mode, as described herein.

The gasification reactor converts a biomass feed and oxygen feed to a syngas stream. The gasification reactor has at least one inlet for the biomass feed and oxygen feed, an outlet for the syngas stream, and a reaction chamber in which the gasification process occurs. Suitable gasification reactors are known in the art and include entrained flow gasifiers and fluidised bed gasifiers. Within the gasification reactor biomass is gasified in the presence of oxygen to produce syngas (a mixture of $H_2$, $CO$ and $CO_2$). It will be understood that the gasification reactor converts biomass, preferably solid biomass, to syngas in a combustion process. A gasification reactor is distinct from a natural gas reformer, a natural gas reformer being provided with a natural gas feed comprising methane and producing hydrogen in a thermal process such as steam-methane reformation or partial oxidation. The syngas exits the gasification reactor as the syngas stream. The gasification reactor produces syngas with high efficiency. Optionally, the gasification reactor is supplied with a biomass feed in the form of solid biomass (as will be described in further detail herein). Optionally, the gasification reactor is operated at a pressure in the range of from about 5 bar to about 60 bar. Optionally, the gasification reactor is operated at a temperature in the range of from about 160 °C to about 400 °C.

Slag is produced as a byproduct of the gasification process. Optionally, slag may be discharged to a slag removal unit. In the slag removal unit, the slag is cooled and discharged in batches from the gasifier bottom. A portion of the slag may be collected and directed to a fine grinding unit and/or mixed with torrefied material as described herein.

For efficient methanol synthesis, the stoichiometric number (Eq. 3) of the syngas fed to the methanol synthesis reactor is ideally a value in the range of about 2.0-2.1. The raw syngas stream produced by the gasification reactor is typically below stoichiometric in hydrogen. Therefore, the $H_2$, CO and $CO_2$ content of the syngas stream must be adjusted so that the syngas stream delivered to the first methanol synthesis reactor has a stoichiometric number in the range of about 2.0-2.1. The system has an overall hydrogen demand defined as the amount of hydrogen needed to adjust the stoichiometric number of the syngas to be in the range of about 2.0-2.1. The stoichiometric number of the raw syngas may be adjusted by increasing the hydrogen content of the syngas. The hydrogen demand of the system may be met by passing at least a first portion of the syngas stream to the water-gas shift reactor to form a hydrogen-rich syngas. Additionally or alternatively, the hydrogen demand of the system may be met by generating hydrogen using the electrolysis unit and providing the hydrogen to the methanol synthesis reactor, as described herein. When a hydrogen stream is provided to the methanol synthesis reactor by the electrolysis unit, the hydrogen demand needed to be met by the water-gas shift reactor is reduced. When the electrolysis unit is in operation, at least a portion of the syngas stream passes through the bypass conduit so that it is not passed through the water-gas shift reactor.

The air-separation unit separates oxygen from atmospheric air and supplies oxygen to the gasification reactor as at least a portion of the oxygen feed. Suitable air-separation units are known in the art. The air separation unit comprises an air inlet for receiving air and an oxygen outlet for discharging oxygen to the gasification reactor. The outlet for the oxygen stream of the air-separation unit is in fluid communication with the gasification reactor so that oxygen produced by the air-separation unit can be provided as at least a portion of the oxygen feed. The air-separation unit may also separate other components from air, such as nitrogen, for use elsewhere in the system. For example, nitrogen can be used to pressurise the biomass feed before the biomass feed is fed into the gasification reactor. Optionally, the air-separation unit has a maximum output of at least 55 t/h (tonnes per hour) of oxygen. It will be understood that the oxygen output of the air-separation unit may be adjustable between a maximum and minimum output. Optionally, the oxygen output and energy demand of the air-separation unit is determined by the oxygen demand of the gasification reactor. Typically, the power demand of the air-separation unit is from about 25 MW (in case of fluidised bed gasifier) up to about 45 MW (in case of entrained flow gasifier), due to the different oxygen operating pressure requirements. The system may comprise multiple air separation units for providing oxygen to the gasification reactor. The system may be operated with or without the air separation unit or units engaged, for example in response to green electricity supply or cost.

The electrolysis unit splits water into oxygen and hydrogen. The electrolysis unit comprises multiple electrolysis cells arranged in stacks for splitting water into hydrogen and oxygen. Optionally, the electrolysis cells are alkaline electrolysis cells. Suitable electrolysis units and cells are known in the art. The electrolysis cell may for example use nickel electrodes at temperatures below 100 °C to split water from a strongly alkaline electrolyte (typically over 20 wt% KOH). Optionally, each electrolysis cell may have a power demand of around 1-3 MW and the cells may be coupled together in stacks of up to 100MW, such as stacks of 20-25 MW. Multiple stacks may be employed.

The system may comprise multiple electrolysis units. Additional electrolysis units may be added to expand the capacity of the system. For example, additional electrolysis units may be added to provide additional green hydrogen to increase methanol production.

Each electrolysis unit comprises a water inlet, an oxygen outlet for discharging oxygen as an oxygen stream and a hydrogen outlet for discharging a hydrogen stream. The oxygen outlet of the electrolysis unit is in fluid communication with the gasification reactor so that oxygen produced by the electrolysis unit can be provided as at least a portion of the oxygen feed. The hydrogen outlet of the electrolysis unit is in fluid communication with the methanol synthesis reactor so that the hydrogen stream may be delivered to the methanol synthesis reactor.

Optionally, the power requirement of the electrolysis unit when the system is operated in full-carbon capture mode is about 800-1200 MW, for example 900-1000 MW. In full-carbon capture mode, the hydrogen output of the electrolysis unit or units is equal to or exceeds the hydrogen demand of the system and surplus oxygen is produced by the electrolysis unit. The power requirement of electrolysis unit needed to replace the air-separation unit (i.e. to meet the oxygen output of the air-separation unit) is optionally 426 MW. It will be understood that the oxygen and hydrogen outputs of the electrolysis unit may be adjustable between a maximum and minimum output.

The water-gas shift reactor receives at least a portion of syngas stream from the gasification reactor and converts the syngas to a hydrogen-rich syngas. Within the reactor the at least a portion of syngas received from the gasification reactor is treated in water-gas shift process reactor to adjust the $H_2$, $CO_2$ and CO concentration of the syngas. In the water-gas shift process, carbon monoxide and water (steam) are converted to carbon dioxide and hydrogen (Eq. 4) to increase the hydrogen content of the syngas, thus providing a hydrogen-rich syngas. Optionally, the water-gas shift reactor also converts organic sulphur components within the raw syngas to hydrogen sulphide for subsequent desulphurization in the acid-gas removal unit. The water-gas shift reactor is located downstream of the gasification reactor and upstream of the methanol synthesis reactor. Suitable water-gas shift reactors are known in the art. Known water-gas shift catalysts are employed in the water-gas shift reactor and include cobalt-molybdenum catalysts and Johnson Matthey K8-11 series water-gas shift catalysts. The water-gas shift reactor has syngas stream inlet for receiving at least a portion of the syngas stream from the gasification reactor and a hydrogen-rich syngas stream outlet for discharging a hydrogen-rich syngas produced by the water-gas shift process. The amount of syngas fed to the water-gas shift reactor is adjustable, for example, in response to the hydrogen output of the electrolysis unit.

Optionally, the gasification reactor, water-gas shift reactor and first methanol synthesis reactor are arranged in series and are fluidly connected by a flow path for directing the first portion of the syngas from the gasification reactor to the methanol synthesis reactor via the water-gas shift reactor.

Optionally, the system comprises a bypass conduit for at least a portion of the syngas stream from upstream of the water-gas shift reactor to downstream of the water-gas shift reactor. The bypass conduit allows a portion of the syngas stream to be diverted so that the portion of syngas does not pass through the water-gas shift reactor.

Optionally, the system comprises a bypass control system for controlling the flow of syngas to the water-gas shift reactor and the flow of syngas through the bypass conduit. Optionally, the bypass control system comprises one of more control valves for controlling the flow of syngas to the water-gas shift reactor and/or the flow of syngas through the bypass conduit. The one or more control valves are adjustable so that the flow rate of syngas passed through the bypass conduit can be controlled and/or the flow rate of the syngas to the water-gas shift reactor is adjustable.

The bypass conduit and the bypass control system provide flexibility so that the amount of syngas bypassing the water-gas shift reactor can be adjusted. This may be particularly advantageous when hydrogen is supplied by the electrolysis unit to the methanol synthesis reactor. As described, the hydrogen from the electrolysis unit increases the hydrogen content of the combined stream to improve the stoichiometric number of syngas for methanol synthesis. When hydrogen stream is supplied to the methanol synthesis reactor, this reduces or removes the need for syngas to be passed through the water-gas shift reactor to form a hydrogen-rich syngas. As the water-gas shift reactor also converts carbon monoxide to carbon dioxide, reducing the amount of syngas that passes through the water-gas shift reactor increase the amount of carbon that is converted to methanol, improving the efficiency and yield of the process. When a hydrogen stream is provided by the electrolysis unit, at least a portion of syngas may be directed through the bypass conduit and without passing through the water-gas shift reactor. The syngas stream passing through the bypass conduit is mixed with the hydrogen stream provided by the electrolysis unit to achieve the desired stoichiometric number. Bypassing at least a portion of the syngas stream so that it does not pass through the water-gas shift reactor reduces the carbon content of the syngas lost as carbon dioxide.

The portion of syngas stream treated in the water-gas shift reactor may be adjustable. Optionally, the syngas stream produced by the gasification reactor is received by a conduit comprising a splitter for directing at least a portion of the syngas stream to the water-gas shift reactor and at least a portion of the syngas stream through the bypass conduit. Optionally, the bypass control system comprises a three way valve for controlling the flow of syngas to the water gas shift reactor and the flow of syngas through the bypass conduit. Additionally or alternatively, the system may comprise a conduit for delivering syngas from the gasification reactor to the water-gas shift reactor and a separate conduit for delivering syngas from the gasification reactor to the bypass conduit. Optionally, each of the conduits comprise a control valve as part of the bypass control system for controlling the flow of syngas to the water-gas shift reactor and the flow of the second portion of the syngas stream through the bypass conduit. In embodiments, the bypass system comprises a first two-way valve for controlling the flow of at least a portion of syngas to the water-gas shift reactor and a second two-way valve for controlling the flow of at least a portion of syngas through the bypass conduit. The first and second control valves, when present, may be operated independently, but preferably the bypass valve and control valve, when present, are operated simultaneously.

Optionally, the bypass control system is configurable so that all the syngas stream passes through the bypass conduit (e.g. the flow rate of the at least a portion of syngas stream to the water-gas shift reactor is zero). Optionally, the bypass control system is configurable so that none of the syngas stream passes through the bypass conduit (e.g. the flow rate of the at least a portion of the syngas stream through the bypass conduit is zero). It will be understood that bypass control system is configurable to any arrangement in-between the extremes described, such that at least some syngas stream is passed to the water-gas shift reactor and at least some syngas stream is passed through the bypass conduit.

The flow of syngas in each operational mode of the system is described in Table 1. It will be understood that the intermediate mode not a discrete mode, but rather the range of modes between the extremes of gasification-only and full-carbon recovery mode.

**Table 1: Operational modes of the system**

| | Gasification-only mode | Intermediate mode | Full-carbon recovery mode |
|---|---|---|---|
| **Air-separation unit** | Operated (maximum capacity) | Operation dependent on electrolysis unit output | Not operated |
| **Electrolysis unit** | Not operated | Operation dependent on air-separation unit output | Operated (maximum capacity) |
| **Bypass conduit** | No syngas through bypass conduit | >0% and <100% syngas through bypass conduit | All syngas through bypass conduit |

The first methanol synthesis reactor converts hydrogen, carbon monoxide and carbon dioxide to methanol in exothermic reactions Eq.1 and Eq. 2. The first methanol synthesis reactor is located downstream of the water-gas shift reactor and bypass conduit. Suitable methanol synthesis reactors are known in the art. Optionally, the first methanol synthesis reactor is an isothermal methanol synthesis reactor comprising multi-tube reactors with boiling water as the cooling fluid. Suitable catalysts for the methanol synthesis process include $Cu/ZnO/Al_2O_3$ catalysts. Optionally, the methanol synthesis reactor is operated at a temperature in the range of about 200 °C and about 280 °C. Optionally, the methanol synthesis reactor is operated at a pressure in the range of from about 50 bar to about 90 bar, such as about 70 bar.

Optionally, the first methanol synthesis reactor comprises at least one inlet for receiving at least one of the hydrogen-rich syngas stream, the second portion of the syngas stream passed through the bypass conduit and the hydrogen stream from the electrolysis unit. Optionally, the at least one of the hydrogen-rich syngas stream, the second portion of the syngas stream passed through the bypass conduit and the hydrogen stream from the electrolysis unit are delivered as a combined stream to an inlet of the first methanol synthesis reactor. Additionally or alternatively, the first methanol synthesis reactor comprises a first inlet for receiving the hydrogen-rich syngas from the water-gas shift reactor, a second inlet for receiving the second portion of syngas stream passed through the bypass conduit, and a third inlet for receiving the hydrogen stream from the electrolysis unit independently of one another. In such an arrangement, the hydrogen-rich syngas stream, the second portion of syngas stream bypassed via the bypass conduit and the hydrogen stream from the electrolysis unit are combined within the methanol synthesis reactor to form a combined stream. Alternatively, the first methanol synthesis reactor comprises an inlet for receiving a combined stream of the hydrogen-rich syngas stream, the second portion of syngas stream bypassed via the bypass conduit and the hydrogen stream from the electrolysis unit that have been combined into a single combined stream upstream of the methanol synthesis reactor.

In embodiments, the gasification reactor is an entrained flow gasifier. Optionally, the entrained flow gasifier has a maximum syngas stream output of at least 300 t/h. Optionally, the entrained flow gasifier operates at a pressure of between about 35 and 45 bar and a temperature of between about 180 °C and 240 °C. Suitable entrained flow gasifiers are known in the art. The entrained flow gasifier produces a high pressure steam as a product of the gasification process. Optionally, the high pressure steam is used elsewhere in the system e.g. for providing heat or mechanical work, as described herein. Advantageously, when the gasification reactor is an entrained flow gasifier, a hot oxygen burner system and partial oxidation reactor is not needed for the destruction of tars. Preferably, the biomass is conditioned before being fed to the entrained flow gasifier, for example as described in relation to the fifth and sixth aspects of the invention.

In embodiments, the gasification reactor is a fluidised bed gasifier. Optionally, the fluidized bed gasifier has a maximum syngas stream output of at least 80 t/h. Optionally, the fluidized bed gasifier operates at a pressure of between about 7 and 50 bar and a temperature of between about 310 °C and 380 °C. The fluidised bed reactor is continuously fed with bed material such as dolomite and/or kaoline. Suitable fluidized bed gasifiers are known in the art. Superheated steam is consumed in the gasification process of fluidised bed gasification reactor. Optionally, the fluidised bed gasifier further comprises a hot oxygen burner and partial oxygen reactor for the destruction of tars. Optionally, the fluidised bed gasifier further comprises a heat exchange system for cooling the syngas stream exiting the gasifier. The heat exchange system produces a high pressure steam for use elsewhere in the system, e.g. for providing heat or mechanical work, as described herein. Advantageously, the fluidised bed gasifier does not require torrefied biomass. For example, the fluidised bed gasifier can operate with a biomass feed in the form of wood chips and/or wood pellets.

In embodiments, the system comprises multiple gasification reactors. To meet the syngas demand of the system, multiple fluidised bed gasifiers may be provided instead of, or in combination with, the entrained flow gasifier. Optionally, the system comprises a combination of at least one entrained flow gasifier and at least one fluidized bed gasifier. Advantageously, the number of reactors in operation may be adjusted in response to the biomass throughput of the system.

In embodiments, the system comprises a distillation column for purifying the crude methanol produced by the methanol synthesis reactor or reactors and producing a methanol distillate. Optionally, the first distillation column comprises an inlet for receiving methanol effluent from the first methanol synthesis reactor and an outlet for discharging a methanol distillate having a higher content of methanol by weight % than the methanol effluent. Optionally, the methanol distillate has a methanol content of at least 99.85 wt% methanol (AA grade methanol) suitable distillation columns are known to the skilled person. Optionally, heat required for the distillation column is provided by a central heat recovery system as described herein.

In embodiments, the system comprises a second and a third methanol synthesis reactor for converting syngas to methanol. Optionally, the first methanol synthesis reactor and the second methanol synthesis reactor are arranged in parallel so that both of the first and second methanol synthesis reactors are arranged to receive:

(iii) the hydrogen-rich syngas from the hydrogen-rich syngas stream outlet of water-gas shift reactor; and/or,
(iv) the syngas from the bypass conduit and hydrogen from the hydrogen outlet of the electrolysis unit.

Optionally, the first and second methanol synthesis reactor, when present, comprise: an outlet for discharging a methanol effluent. Optionally, each of the first and second methanol synthesis reactors have an outlet for discharging unreacted syngas. Optionally, the first and second methanol synthesis reactors have a capacity such that when the system is operated in the gasification-only mode, all syngas is converted by the first and second methanol synthesis reactors. However, in full-carbon recovery mode or an intermediate mode, the combined syngas input may exceed the capacity of the first and second methanol synthesis reactors and unreacted syngas is discharged from the first and second methanol synthesis reactors as an unreacted syngas. Optionally, the system further comprises a third methanol synthesis reactor for receiving unreacted syngas from the first and/or second methanol synthesis reactors. Optionally, the third methanol synthesis reactor is arranged in series with the first and/or the second methanol synthesis reactor. Optionally, the third methanol synthesis reactor comprises an inlet for receiving the unreacted syngas from at least one of the first and second methanol synthesis reactors and an outlet for discharging a methanol effluent. Thus, a system comprising at least three methanol reactors provides flexibility to operate the system in the various modes described.

In embodiments, the system comprises a second and third methanol distillation column for producing a methanol distillate. Optionally, the first methanol distillation column receives crude methanol (methanol effluent) from the first methanol synthesis reactor. Optionally, the second methanol distillation column receives crude methanol (methanol effluent) from the second methanol synthesis reactor. Optionally, the third methanol distillation column receives crude methanol (methanol effluent) from the third methanol synthesis reactor. Optionally, first methanol distillation column comprises an inlet for receiving the methanol effluent from the first methanol synthesis reactor. Optionally, the second methanol distillation column has an inlet for receiving the methanol effluent from the second methanol synthesis reactor. Optionally, the third methanol distillation column has an inlet for receiving the methanol effluent from the third methanol synthesis reactor. Optionally, each of the first, second and third methanol distillation columns comprise an outlet for discharging a methanol distillate having a higher content of methanol by weight % than the methanol feed entering the inlet. Optionally, the methanol distillate of each methanol distillation column has a methanol content of at least 99.85 wt% methanol (AA grade methanol).

In embodiments, the system comprises an acid gas removal unit for removing acid gases such as carbon dioxide and $H_2S$ from syngas. It is advantageous to remove acid gases from the syngas as these may poison the catalyst of the methanol synthesis reactor. Furthermore, the acid-gas removal unit may be used to remove carbon dioxide to adjust the stoichiometric number of the syngas. At least a portion of carbon dioxide recovered by the acid-gas removal unit may be used in the gasification unit for purging, sluicing and/or feeding, as described herein. Any excess carbon dioxide may be vented to the atmosphere as biogenic carbon dioxide or stored. The acid-gas removal system, if present, is located downstream of the water-gas shift unit and upstream of the first methanol synthesis reactor. Suitable acid-gas units are known in the art. Optionally, the acid gas removal unit has an inlet for receiving hydrogen-rich syngas from the water-gas shift reactor, and optionally syngas from the bypass conduit; an absorber/desorber system for acid gas recovery; an outlet for discharging recovered acid gas; and an outlet for cleaned syngas. Optionally, the absorber/desorber system comprises an amine-absorber/desorber system and the recovered acid gas comprises carbon dioxide. Optionally, the acid gas removal unit comprises at least two absorber/desorber columns arranged in series to provide the capacity and flexibility needed for the system to operate in any mode. Optionally, the acid-gas removal unit are provided with at least two sulphur guard bed absorbers to remove sulfur gases not captured by the absorber/desorber columns.

In embodiments, the system comprises a scrubber unit for removing impurities from the raw syngas produced by the gasification reactor. For example, the scrubber unit removes HCl, fine particles of dust, halides and liquid aerosols. Optionally, the scrubber unit is located downstream of the gasification reactor and upstream of the water-gas shift reactor and bypass conduit. Optionally, the scrubber unit comprises an inlet for receiving raw syngas from the gasification reactor and an outlet for discharging the syngas stream that has been scrubbed.

Optionally, the air-separation unit and the electrolysis unit of the system are located at the same site as the other components of the system, and in particular the gasification reactor, water-gas shift reactor and first methanol synthesis reactor. Alternatively, the air-separation unit and electrolysis unit may be located at a different site. The off-site air-separation unit and electrolysis unit may be connected to the gasification reactor by one or more pipelines to deliver hydrogen and/or oxygen to site. Additionally or alternatively, the hydrogen from the electrolysis unit and the oxygen from the electrolysis unit and/or air-separation unit may be collected from the electrolysis unit and/or the air-separation unit, for example in cryogenic tankers, and delivered to site and supplied to the gasification reactor. In such embodiments, the system does not comprise the air-separation unit or the electrolysis unit but instead comprises a first oxygen conduit for delivering oxygen extracted from air using an air-separation to the gasification reactor as at least a portion of the oxygen feed a second oxygen conduit for delivering oxygen produced by an electrolysis unit to the gasification reactor to provide the oxygen as at least a portion of the oxygen feed; and a first hydrogen conduit for delivering hydrogen. All other components of the system in such embodiments are as described herein.

It will be understood that embodiments described in relation to the first aspect of the invention may include any feature described in relation to any other aspect of the invention. For example, the system of the first aspect of the invention may include a drying unit or torrefaction unit according to the sixth and seventh aspects of the invention, and/or a central heat recovery system according to the fourth and fifth aspects of the invention.

According to a second aspect of the invention is provided a method for converting biomass to methanol in a system comprising

a gasification reactor;
a water-gas shift reactor located downstream of the gasification reactor;
a first methanol synthesis reactor located downstream of the water-gas shift reactor;
a bypass conduit for diverting syngas from upstream of the water-gas shift reactor to downstream of the water-gas shift reactor and without passing through the water-gas shift reactor;
optionally a bypass control system for controlling the flow of syngas to the water-gas shift reactor and the flow of syngas through the bypass conduit;
an air-separation unit for supplying oxygen to the gasification reactor; and,
an electrolysis unit for supplying oxygen to the gasification reactor and hydrogen to a first methanol synthesis reactor.

The method according to the second aspect of the invention comprises

converting a biomass feed and an oxygen feed to a syngas stream in the gasification reactor;
feeding at least a portion of the syngas stream to the water-gas shift reactor to produce a hydrogen-rich syngas stream;
operating the electrolysis unit to produce a hydrogen stream;
feeding at least a portion of the syngas stream through the bypass conduit and without passing through the water-gas shift reactor;
combining the hydrogen-rich syngas stream, syngas from the bypass conduit and the hydrogen stream to form a combined stream; and,
converting the combined stream to methanol in the first methanol synthesis reactor.

Optionally, the method further comprises:

adjusting the hydrogen stream output of the electrolysis unit; and,
operating the bypass control system to maintain the stoichiometric number of the combined stream within a predetermined range, wherein the stoichiometric number (SN) is defined as:

$$SN = ([H_2] - [CO_2])/([CO] + [CO_2])$$

and $[H_2]$ is the hydrogen concentration of the combined stream, $[CO_2]$ is the carbon dioxide concentration of the combined stream and $[CO]$ is the carbon monoxide concentration of the combined stream.

Preferably, the predetermined range is 2.0-2.1. The hydrogen output of the electrolysis unit can be adjusted in response to green electricity supply and cost. As a consequence of adjusting the hydrogen output of the electrolysis unit, the stoichiometric number of the combined stream will vary according to Eq. 3. When the stoichiometric number of the combined stream lies outside or is trending towards a value outside of the desired stoichiometric range, referred to as the predetermined stoichiometric range, the bypass control system is operated to maintain the stoichiometric number of the combined stream within the predetermined range, preferably wherein the predetermined range is 2.0-2.1. It will be understood that operating the bypass control system to maintain the stoichiometric number of the combined stream may comprise adjusting the flow of syngas to the water-gas shift reactor and/or adjusting the flow of syngas through the bypass conduit so that the stoichiometric number remains within or returns to the predetermined stoichiometric range. For example, when the hydrogen stream output of the electrolysis unit is increased (more hydrogen is produced), more syngas must be bypassed via the bypass conduit to achieve the desired stoichiometric number of the combined stream. The bypass control system increases the flow of second portion of syngas through the bypass conduit to be mixed with the hydrogen stream. In contrast, when the hydrogen stream output of the electrolysis unit is decreased (less hydrogen is produced), less syngas must be bypassed via the bypass conduit to achieve the desired stoichiometric number of the combined stream. The bypass control system decreases the flow of second portion of syngas through the bypass conduit to be mixed with the hydrogen stream.

In embodiments, the method further comprises operating the bypass control system so that all of the syngas stream passes through the water-gas shift reactor to form the hydrogen-rich syngas, and converting the hydrogen-rich syngas stream to methanol in the first methanol synthesis reactor. In embodiments, the method further comprises operating the bypass control system so that all of the syngas stream passes through the bypass conduit, and converting the syngas from the bypass conduit and the hydrogen stream from the electrolysis unit to methanol in the first methanol synthesis reactor.

Optionally, the method further comprises operating the air separation unit and/or the electrolysis unit to supply oxygen to the gasification reactor. Thus, in embodiments, the air-separation unit supplies at least a portion of the oxygen feed of the gasification reactor and the electrolysis unit also supplies at least a portion of the oxygen feed of the gasification reactor, and the method further comprises adjusting the flow of oxygen from the air-separation unit to the gasification reactor and the flow of oxygen from the electrolysis unit to the gasification reactor in response to the adjustment of the hydrogen stream output of the electrolysis unit. Optionally, when the hydrogen stream output of the electrolysis unit is increased, the method further comprises increasing the flow of oxygen from the electrolysis unit to the gasification reactor and decreasing the flow of oxygen from the air-separation unit to the gasification reactor. Optionally, when the hydrogen stream output of the electrolysis unit is decreased, the method further comprises decreasing the flow of oxygen from the electrolysis unit to the gasification reactor and increasing the flow of oxygen from the air-separation unit to the gasification reactor.

Optionally, the stoichiometric number of the combined stream is measured continuously or periodically. Optionally, the stoichiometric number of the combined stream may be measured upstream of the first methanol synthesis reactor and downstream of the water-gas shift reactor such as at the inlet of the first methanol synthesis reactor for receiving the combined stream.

In embodiments, the system may optionally comprise a sample outlet for venting a sample of the combined stream and a detector or device for determining the stoichiometric number of the sample of the combined stream. Such outlet may be located upstream of the first methanol synthesis reactor and downstream of the water-gas shift reactor such as at the inlet of the first methanol synthesis reactor for receiving the combined stream.

Optionally, the bypass control system comprises a controller and at least one control valve. Optionally, operating the bypass control system to maintain the stoichiometric number of the combined stream within a predetermined range may comprise:

determining the stoichiometric number of a sample of the combined stream vented from the sample outlet using the detector;
providing the stoichiometric number of the combined stream as a data input into the controller;
comparing the stoichiometric number of the combined syngas to the predetermined range stored on the controller as a reference stoichiometric range and,
when the stoichiometric number of the combined stream is outside of the reference stoichiometric range, adjusting the at least one control valve to adjust the flow of the syngas to the water-gas shift reactor and/or the flow of syngas through the bypass conduit.

The controller allows adjustment of the control valve to be automated. The sample outlet is located at a position that the combined stream can be vented prior to the combined stream being converted to methanol in the methanol synthesis reactor. In embodiments, the step of monitoring the stoichiometric number of the combined stream may further comprise determining the stoichiometric number of a sample of the combined stream vented from the sample outlet using the detector; providing the stoichiometric number of the combined stream as a data input into the controller; comparing the stoichiometric number of the combined syngas to a reference stoichiometric range stored in the controller, optionally, wherein the reference stoichiometric range is 2.0-2.1; and, when the stoichiometric number of the combined stream is outside of the reference stoichiometric range, the step of adjusting the position of the at least one control valve is initiated by the controller.

The controller may comprise software or other machine-readable instructions that when executed cause the controller to change the configuration of the at least one control valve, if present. The controller may comprise one or more processors or other conventional computing hardware on which such software or machine-readable instructions can be executed. The controller may comprise electronic control circuitry of the type known in the art to enable the execution of said software or other machine-readable instructions to cause changes in the position of the at least one control valve, if present. A reference stoichiometric range may be stored in a database of the controller. Optionally, the reference stoichiometric range is about 2.0-2.1. The controller may receive the stoichiometric number of the combined stream as determined by the detector as a data input and compare the data input to the reference stoichiometric range. When the stoichiometric number of the combined stream is outside the reference stoichiometric range, the controller may adjust the position of the at least one control valve.

Optionally, the stoichiometric number of the hydrogen-rich syngas stream is measured continuously or periodically in addition to or instead of the stoichiometric number of the combined stream. For example, the stoichiometric number of the hydrogen-rich syngas stream may be measured at or downstream of the hydrogen-rich syngas stream outlet of the water-gas shift reactor. Optionally, the system may comprise a sample outlet for venting a sample of the hydrogen-rich syngas stream and a detector or device for determining the stoichiometric number of the sample of the hydrogen-rich syngas stream, the detector or device being the same or different to the detector or device for determining the stoichiometric number of the sample of the combined stream. Such sample outlet may be located at the hydrogen-rich syngas stream outlet of the water-gas shift reactor or downstream of said outlet. The sample outlet may be provided in addition to or instead of the sample outlet for venting a sample of the combined stream. The sample outlet allows the stoichiometric number of the hydrogen-rich syngas stream to be continuously or periodically monitored. Because the hydrogen-rich syngas stream forms at least a portion of the combined stream, the controller of the bypass control system may adjust the position of control valve in response to the stoichiometric number of the hydrogen-rich syngas stream so that the combined steam has a stoichiometric number within the predetermined range. Optionally, the stoichiometric number of the hydrogen-rich syngas stream may be provided as a data input into the controller. The stoichiometric number of the hydrogen-rich syngas stream may be provided as a data input into the controller instead or in addition to the stoichiometric number of the combined stream. The configuration of the at least one control valve of the controller may be adjusted in response to said data input. For example, if the stoichiometric number of the hydrogen-rich syngas is outside of the predetermined range, then the at least one control valve may be adjusted to increase or decrease the flow of syngas fed to the water-gas shift reactor, and/or the flow of syngas through the bypass conduit. Thus, operating the bypass control system to maintain the stoichiometric number of the combined stream within a predetermined range may comprise:

determining using the detector the stoichiometric number of a sample of the hydrogen-rich stream vented from a sample outlet for venting a sample of the hydrogen-rich syngas stream;

providing the stoichiometric number of the hydrogen-rich syngas stream as a data input into the controller;

comparing the stoichiometric number of the hydrogen-rich syngas stream to the predetermined range stored on the controller as a reference stoichiometric range and,

when the stoichiometric number of the hydrogen-rich stream is outside of the reference stoichiometric range, adjusting the at least one control valve to adjust the flow of the syngas to the water-gas shift reactor and/or the flow of syngas through the bypass conduit. Optionally, the flow rate of the hydrogen stream output of the electrolysis unit may be adjusted instead of or in addition to the flow rates of the syngas to the water-gas shift reactor and/of the syngas through the bypass conduit.

In embodiments, the system used in the method of the second aspect of the invention is a system according to any other aspect of the invention, preferably a system according to the first aspect of the invention.

According to a third aspect of the invention is provided a system for converting biomass to methanol in which heat from various components of the system is recovered and distributed to other components of the system requiring heat. The system according to the fourth aspect of the invention comprises:

a drying unit for drying raw biomass to produce a dried biomass feed;

a gasification reactor for converting the dried biomass feed and an oxygen feed to syngas;

a water-gas shift reactor for increasing the hydrogen content of the syngas to form a hydrogen-rich syngas stream;

an acid gas removal unit for removing acidic gases from the hydrogen-rich syngas stream;

a first methanol synthesis reactor for converting the hydrogen-rich syngas to crude methanol; and,a methanol distillation column for purifying the crude methanol to produce a methanol distillate;wherein each of the gasification reactor and first methanol synthesis reactor comprise a heat exchange system for controlling the temperature of the gasification reactor and/or methanol synthesis reactor and producing steam using recovered heat; and,

each of the drying unit, acid-gas removal unit and methanol distillation column comprise a heat exchange system for transferring heat from the steam to the drying unit, acid-gas removal unit or methanol distillation column.

Optionally, the system further comprises a central heat recovery system for receiving the steam from the heat exchange system of the gasification reactor and first methanol synthesis reactor and delivering the steam to the heat exchange system of each of the drying unit, acid-gas removal unit and methanol distillation column. It will be understood that the system may comprise any feature described in relation to any other aspect of the invention. For example, the drying unit, gasification reactor, water-gas shift reactor, acid gas removal unit, first methanol synthesis reactor, and methanol distillation column may be according to the equivalent components described in relation of the first and second and third aspects of the invention.

Prior to the present invention, systems for producing methanol from biomass typically dissipate waste heat from the process to the atmosphere or use the waste heat to generate steam for producing electricity. The present system provides a central heat recovery system for receiving steam produced by recovery of heat from at least one of the gasification reactor and methanol synthesis reactor. The central heat recovery system distributes the steam to at least one of the drying unit, acid gas removal unit and methanol distillation column to provide heat to the processes carried out in those systems. Optionally, none of the steam generated in the process is used to generate electricity.

The gasification reactor, water-gas shift reactor, acid-gas removal unit, first methanol synthesis reactor and methanol distillation column are as described according to the first and second aspects of the invention. The drying unit is for removing water from the raw biomass feed delivered to the system. Typically, raw biomass has a water content in the region of 40-50 wt% based on the weight of the biomass. Optionally, the water content of the biomass leaving the drying unit is less than 20 wt %. Optionally, the drying unit dries the biomass by passing heated air through a biomass bed. A heat exchange system may be used to transfer heat to an air stream to produce hot air for drying. Suitable drying units are known in the art. Optionally, the water-gas shift reactor comprises a heat exchange system for cooling the hydrogen-rich syngas stream produced by the water-gas shift reactor. Optionally, the heat exchange system of the water-gas shift reactor is integrated with the central heat recovery system.

Optionally, one or more of the drying unit, gasification reactor, water-gas shift reactor, acid-gas removal unit, first methanol synthesis reactor and methanol distillation unit each have a heat exchange system integrated with the central heat recovery system. The heat exchange system transfers heat from a heat source (such as a reactor) to a heat transfer medium (such as water or steam). A heat exchange system may be used to remove heat from the methanol synthesis reactor and methanol distillation unit. A heat exchange system may be used to provide heat to the drying unit, water-gas shift reactor, acid-gas removal unit and methanol distillation column. The central heat recovery system allows steam to be distributed around the system. Suitable heat exchange systems are known in the art, for example cooling jackets, cooling coils, condensers, evaporators and cooling towers.

In embodiments, the heat exchange system of the gasification reactor produces a high pressure steam having a pressure of at least 45 bar and a temperature of at least 255 °C. Optionally, the high pressure steam has a pressure of at least 50 bar and a temperature of at least 265 °C. Optionally, the central heat recovery system comprises a high pressure steam section for receiving the high pressure steam and delivering the high pressure steam to the heat exchange system of the acid-gas removal unit.

In embodiments, the heat exchange system of the first methanol synthesis reactor produces a medium pressure steam having a pressure of greater than or equal to 25 bar and less than 45 bar and a temperature of greater than or equal to 220 °C and less than 255 °C. Optionally, the medium pressure steam has a pressure of between about 25 and 35 bar and a temperature of between about 225 °C and 240 °C. Optionally, the central heat recovery system comprises a medium pressure steam section for receiving the medium pressure steam and delivering the medium pressure steam to the heat exchange system of the methanol distillation column.

In embodiments, the central heat recovery system comprises a cascading stage between the high pressure steam section and medium pressure steam section for expanding a portion of the high pressure steam of the high pressure section to a medium pressure steam. Optionally, the central heat recovery system comprises a cascading stage between the medium pressure steam section and a low pressure steam section for expanding a portion of the medium pressure steam of the medium pressure section to a low pressure steam. Optionally, the low pressure steam has a pressure of greater than or equal to 4 bar and less than 25 bar and a temperature of greater than or equal to 140 °C and less than 220 °C. Optionally, the low pressure steam has a pressure of greater than or equal to 5 bar and less than 10 bar and a temperature of greater than or equal to 150 °C and less than 170 °C. Optionally, the central heat recovery system delivers low pressure steam to the heat exchange system of the methanol distillation column and the drying unit.

In embodiments, the system comprises at least one of:

an oxygen compressor upstream of the gasification reactor for increasing the pressure of the oxygen feed,
a syngas compressor upstream of the methanol synthesis reactor for increasing the pressure of the hydrogen-rich syngas,
a hydrogen compressor upstream of the methanol synthesis reactor for increasing the pressure of a hydrogen feed prior to the hydrogen feed entering the methanol synthesis reactor, and
a carbon dioxide compressor for pressurising carbon dioxide discharged from the acid-gas removal unit.

Optionally, at least one of the oxygen compressor, syngas compressor, hydrogen compressor and carbon dioxide compressor comprises a heat exchange system for recovering hot water and/or condensate and providing the hot water and/or condensate to a hot water and/or condensate section of the central heat recovery system. The hot water and/or condensate may be used elsewhere within the system to provide heat. Optionally, the central heat recovery system delivers hot water and/or condensate from the hot water and/or condensate section back to the heat exchange system of at least one of the oxygen compressor, syngas compressor, hydrogen compressor and carbon dioxide compressor. Thus, the hot water and/or condensate used to provide heat elsewhere in the system may be re-heated in the heat exchange system of at least one of the oxygen compressor, syngas compressor, hydrogen compressor and carbon dioxide compressor. Optionally, the heat exchange system of the drying unit comprises a condenser to recover heat from water vapour obtained from drying the biomass. Optionally, the hot water and/or condensate section of the central heat recovery system receives hot water and/or condensate from at least one of the heat exchange system of the acid gas removal unit, methanol distillation column and the drying unit. Optionally, the hot water and/or condensate may be directed to the optional scrubbing unit for use as a scrubbing liquid.

In embodiments, the system optionally further comprises at least one steam turbine for expanding steam supplied by the central heat recover system to produce shaft power for mechanical work. Optionally, the at least one steam turbine produces shaft power to drive at least one of the oxygen compressor, syngas compressor, hydrogen compressor and carbon dioxide compressor.

According to a fourth aspect of the invention, is a method of recovering heat from the system of the third aspect of the invention. The method comprises:

maintaining the temperature of the gasification reactor and first methanol synthesis reactor using the heat exchange system of the gasification reactor and/or methanol synthesis rector and producing steam using the recovered heat;
delivering the steam to the central heat recovery system;
directing steam from the central heat recovery system to the heat exchange system of each of the drying unit, acid-gas removal unit and methanol distillation column to heat the drying unit, acid-gas removal unit and methanol distillation column. The fourth aspect of the invention may include or combine any feature disclosed in relation to any other aspect of the invention.

Optionally, the method comprises generating a high pressure steam having a pressure of at least 45 bar and a temperature of at least 255 °C using the heat exchange system of the gasification reactor and transferring the high pressure steam to a high pressure steam section of the central heat recovery system for distribution to the heat exchange system of the acid-gas removal unit.

Optionally, the method comprises generating a medium pressure steam having a pressure of greater than or equal to 25 bar and less than 45 bar and a temperature of greater than 220 °C and less than 255 °C using the heat exchange system of the first methanol synthesis reactor and transferring the medium pressure steam to a medium pressure steam section of the central heat recovery system for distribution to the heat exchange system of the methanol distillation column.

Optionally, the method comprises in first cascading stage of the central heat recovery system, expanding a portion of the high pressure steam of the high pressure section to form a medium pressure steam and delivering the medium pressure steam to the medium pressure section of the central heat recovery system. Optionally, the method comprises in second cascading stage of the central heat recovery system, expanding a portion of the medium pressure steam of the medium pressure section to form a low pressure steam and delivering the low pressure steam to the low pressure section of the central heat recovery system. Optionally, the low pressure steam has a pressure of greater than or equal to 4 bar and less than 25 bar and a temperature of greater than or equal to 140 °C and less than 220 °C. Optionally, the method further comprises delivering low pressure steam from the low pressure section of the central heat recovery system to the heat exchange system of the methanol distillation column and the heat exchange system of the drying unit.

Optionally, the method comprises recovering hot water and/or condensate from the heat exchange system of at least one of the oxygen compressor, syngas compressor, hydrogen compressor and carbon dioxide compressor and providing the hot water and/or condensate to a hot water and/or condensate section of the central heat recovery system. Optionally, the method further comprises delivering hot water and/or condensate from the hot water and/or condensate section of the central heat delivering system to the heat exchange system of at least one of the oxygen compressor, syngas compressor, hydrogen compressor and carbon dioxide compressor. Optionally, the method comprises recovering hot water and/or condensate from the heat exchange system of at least one of the oxygen compressor, syngas compressor, hydrogen compressor and carbon dioxide compressor and delivering the recovered hot water/condensate to the hot water/condensation section of the central heat recovery system.

Optionally, the method comprises expanding steam supplied by the central heat recovery system in at least one turbine to provide shaft power for mechanical work. Optionally, the at least one steam turbine produces shaft power to drive at least one of the oxygen compressor, syngas compressor, hydrogen compressor and carbon dioxide compressor.

According to a fifth aspect of the invention there is provided a method of converting biomass to methanol in a system comprising: a drying unit, a torrefaction unit, a gasification reactor and a methanol synthesis reactor. The method comprises:

drying a raw biomass feed in the drying unit to produce a dried biomass feed;

converting the dried biomass feed to a torrefied biomass feed in a torrefaction process in the torrefaction unit;

converting the torrefied biomass feed to a syngas feed in a gasification process in the gasification reactor; and,

converting at least a portion of the syngas feed to methanol in the methanol synthesis reactor. It will be understood that the system may comprise any feature described in relation to any other aspect of the invention. Thus, the drying unit, torrefaction unit, gasification reactor and methanol synthesis reactor may be as described, and have any feature mentioned in relation to, the drying unit, torrefaction unit, gasification reactor and methanol synthesis reactor described in relation to any other aspect of the invention.

Slag is produced as a waste product of the gasification process. Typically, slag from gasification reactors is removed and disposed of in landfill. Optionally, the method of the fifth aspect of the invention comprises recycling at least a portion of the slag with at least one of the raw biomass feed, the dried biomass feed, and the torrefied biomass feed in a location upstream of the gasification reactor. The present inventors have found that slag produced as a byproduct of the gasification process has remaining carbon content that may be converted into syngas in the gasification process in a second, third or further cycle within the system. Thus, at least a portion of the slag produced in the gasification process and being discharged from the gasification reactor may be recycled to upstream of the gasifier and combined with the biomass feed to be passed through the gasification reactor a second or third time or more. This improves the efficiency of the process by increasing the carbon content of the biomass converted to syngas, which is ultimately converted to methanol.

The slag or a portion thereof, may be recycled to any suitable point upstream of the gasification reactor to be combined with at least one of the raw biomass feed, the dried biomass feed and the torrefied biomass feed (collectively referred to as "the biomass feeds"). For example, at least a portion of slag may be combined with the raw biomass feed before the raw biomass feed is fed to the drying unit. Additionally, or alternatively, at least a portion of slag may be combined with the dried biomass feed downstream of the drying unit and upstream of the torrefaction process. Additionally, or alternatively, at least a portion of slag may be combined with the torrefied biomass feed downstream of the torrefaction unit and upstream of the gasification reactor. Preferably, at least a portion of the slag is combined with the torrefied biomass feed downstream of the torrefaction unit and upstream of the gasification reactor. The at least a portion of the slag that is recycled to upstream of the gasification reactor has already been passed through the drying and torrefaction units and therefore does not need to be processed again in the drying and torrefaction units before being recycled to the gasification reactor.

The slag exiting the gasification reactor may be mixed with bed reactor material from the gasification reactor. Recycling at least a portion of the slag which is mixed with bed reactor material from the gasification reactor to a location upstream of the gasification reactor returns bed material to the gasification reactor.

The biomass is conditioned before entering the gasification reactor. This may be particularly advantageous when the gasification reactor is an entrained flow gasifier as described herein. The torrefaction unit converts dried biomass to a torrefied biomass by exposing the biomass to high temperatures e.g. 250-300 °C, to partially devolatise the biomass and weaken the fibrous structure. Optionally, downstream of the torrefaction unit is at least one of a pelletizer unit and a fine grinder, for further processing the torrefied biomass before the biomass is fed to the gasification reactor.

Optionally, the amount of slag that is recycled to upstream of the gasification reactor to be combined with at least one of the raw biomass feed, the dried biomass feed and the torrefied biomass feed, may be controlled within set limits. For example, a first portion of the slag that is recycled may be combined with at least one of the raw biomass feed, the dried biomass feed, and the torrefied biomass feed upstream of the gasification reactor at a rate of 2.5 to 4.0 tonnes of slag per hour, preferably 3.0 to 3.5 tonnes of slag per hour. The remaining portion of the slag leaving the gasification reactor and that is not recycled, may be removed from the system downstream of the gasification reactor. For example, a portion of the slag may be discharged into a collection vessel for storage or disposal.

Slag may be removed from the system to control the total content of slag within the system. The amount of slag that is recycled and the amount of slag that is removed may be adjusted during operation of the system, for example to achieve the desired rate at which the slag is combined with at least one of the biomass feeds. Shortly after system start-up, there may be small quantities of slag within the system and all of the slag or a large proportion of the slag, such as least 80 wt% of the slag, may be recycled to upstream of the gasification reactor for mixing with the biomass feeds.

Over time, slag may build up within the system as biomass is processed in the gasification reactor. When the amount of slag within the system exceeds that needed to achieve the desired flow rate at which the slag is recycled upstream of the gasification reactor, slag is removed from the system. The excess slag may be removed from the system downstream of the gasification reactor. Slag may be removed from the system periodically or continuously to control the amount of slag within the system. The waste slag may be removed from the collection vessel for disposal, for example as landfill.

The raw biomass feed defined in any aspect of the invention may be derived from biomass from the agricultural or forestry industry, Advantageously, the carbon and hydrogen content of forestry industry derived biomass remains substantially constant across biomass sources and therefore the syngas composition produced by the gasification reactor remains substantially constant without requiring modification of the gasification reactor or its operation.

Volatiles are produced in the torrefaction process as a waste product. The volatiles may for example comprise light hydrocarbons such as methane and other volatile gases. The volatiles and an oxygen feed may be combusted in a combustor to generate heat for use elsewhere in the system. This reduces the dependence of fossil fuels for providing heat to the system. Most preferably, the heat generated by the combustion of the volatiles may be used to heat the drying unit for drying raw biomass. The system may comprise at least one heat exchange system for transferring heat from the exhaust stream of the combustor to an air stream to produce a hot air stream. Thus, in embodiments, the torrefaction process produces a volatiles stream and the method further comprises:

directing at least a first portion of the volatiles stream to a combustor and combusting the first portion of the volatiles stream in the combustor to produce a hot exhaust stream;
transferring heat from the hot exhaust stream to an air stream in a heat exchange system to produce a hot air stream; and, supplying at least a portion of the hot air stream to the drying unit to provide heat for drying the raw biomass feed.

Suitable combustors and heat exchanger systems are known in the art. The heat exchange system is supplied with an air stream and the hot exhaust stream from the combustor. It will be understood that the air stream and the exhaust stream are prevented from mixing within the heat exchange system but heat is passed from the hot exhaust stream to the air stream within the heat exchanger to form a hot air stream. At least a portion of the hot air stream may be directed to the drying unit to dry the raw biomass feed.

Optionally, a further portion of the volatiles stream is supplied to the torrefaction unit to be used as a fuel for providing heat for the torrefaction process. Optionally, the second portion of the volatiles stream is supplied to one or more burners or combustors of the torrefaction unit for providing heat to a torrefaction chamber in which the torrefaction process takes place.

The process of drying the raw biomass feed in the drying unit produces water vapour. Heat may be recovered from the system by condensing the water vapour. Thus, in embodiments, heat from water vapour released in the drying of the raw biomass feed may be recovered by condensing the water vapour in a condenser. The recovered heat may be used in the drying process.

According to a sixth aspect of the invention, there is provided a system for converting biomass to methanol in a process according to the sixth aspect of the invention. The system may comprise any feature described in relation to any one other aspect of the invention. The system of the sixth aspect of the invention comprises:

a drying unit for drying a raw biomass feed to produce a dried biomass effluent;
a torrefaction unit for converting the dried biomass effluent to a torrefied biomass effluent in a torrefaction process;
a gasification reactor for converting the torrefied biomass effluent to a syngas stream in a gasification process; and,
a methanol synthesis reactor for converting the syngas stream to methanol.

Optionally, the gasification reactor comprises an outlet for discharging slag produced in the gasification process. Optionally, the system comprises a conduit for directing a portion of the slag from the outlet of the gasification reactor to a position upstream of the gasification reactor for mixing with at least one of the raw biomass feed, the dried biomass effluent, and the torrefied biomass effluent. At least one valve may be used to control the flow of slag through the conduit.

Optionally, the system further comprises an outlet for removing slag from the system as waste slag. The outlet may feed waste slag to a collection vessel for storage. For example, the outlet may be connected to the collection vessel by a conduit. The conduit may comprise a valve for controlling the flow of waste slag through the conduit.

The drying unit, torrefaction unit and gasification reactor are arranged in series with the torrefaction unit being located downstream of the drying unit and upstream of the gasification reactor. It will be understood that further units may be located between and in series with, any one of the drying unit, torrefaction unit and gasification reactor. For example, the system may further comprise a pelletizer unit and grinder unit downstream of the torrefaction unit and upstream of the gasification reactor. The pelletizer unit processes the torrefied biomass into pelletized form. The grinder unit grinds the pelletized biomass to produce a finely ground torrefied biomass for feeding to the gasification reactor. Suitable pelletizer units and grinders are known in the art.

Optionally, the torrefaction unit comprises a volatiles stream outlet for discharging a volatiles stream produced in the torrefaction process. Optionally, the system further comprises a combustor for combusting at least a portion of the volatiles stream to produce a hot exhaust stream and a heat exchanger for transferring heat from the hot exhaust stream to an air stream to produce a hot air stream. Optionally, the drying unit is configured to receive the hot air stream to provide heat for drying the raw biomass feed.

Optionally, the system comprises a condenser for recovering heat from water vapour released in the drying of the raw biomass feed in the drying unit. The condenser comprises a cooling fluid for condensing the water vapour. The heat of condensation is transferred from the water vapour to the cooling medium to increase the temperature of the cooling medium to produce a heated fluid. The heated fluid may be transferred to the drying unit, for example a heat exchanger of the drying unit to provide heat for drying the raw biomass feed. It will of course be appreciated that features described in relation to one aspect of the present invention may be incorporated into other aspects of the present invention. For example, the method of the invention may incorporate any of the features described with reference to the systems of the invention and *vice versa.*

Description of the Drawings

Embodiments of the present invention will now be described by way of example only with reference to the accompanying schematic drawings of which:

Figure 1 is a schematic of a system for converting biomass to methanol according to the invention;
Figure 2 is a schematic of an entrained flow gasifier;
Figure 3 is a schematic of a fluidised bed gasifier;
Figure 4 is a schematic of three methanol synthesis reactors according to an embodiment of the invention, wherein the first and second methanol synthesis reactors are arranged in parallel and the third methanol synthesis reactor is arranged to receive unreacted syngas from both of the first and second methanol synthesis reactor;
Figure 5 is a flow diagram showing a heat recovery system according to the invention;
Figure 6 is a flow diagram showing a process of converting biomass to methanol in the system of figure 1;
Figure 7 is a flow diagram of a process according to the present invention in which slag is recycled to the biomass feed;
Figure 8 is a graph showing the hydrogen output of the electrolysis unit and the amount of syngas passed through the water-gas shift unit in the operational modes of the system of figure 1.

Detailed Description

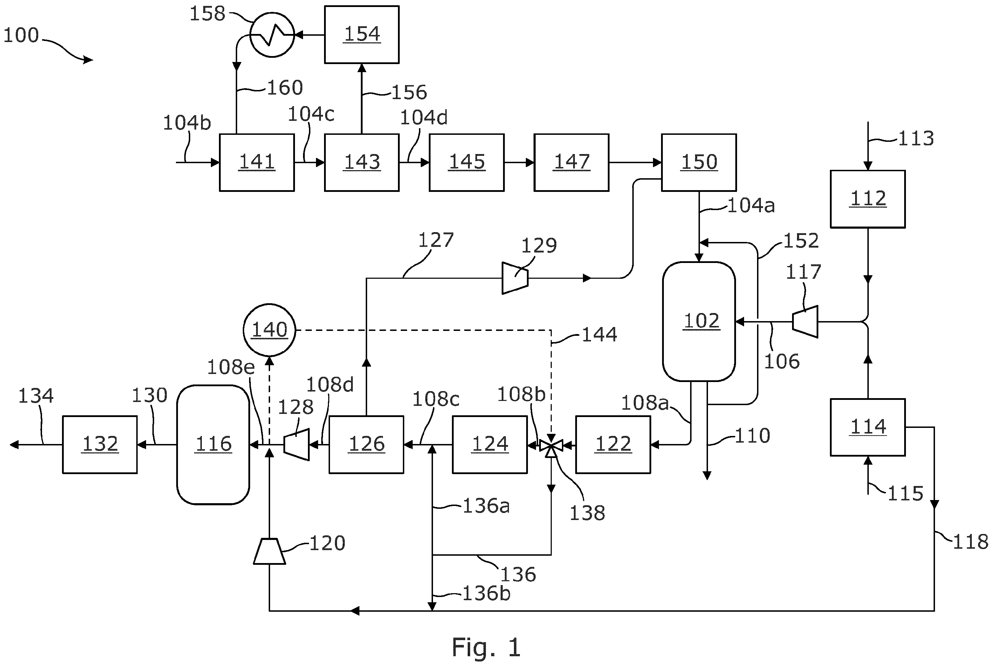

A system 100 according to an embodiment of the invention is shown in Figure 1. The system 100 comprises a gasification reactor 102 for converting a biomass feed 104a and an oxygen feed 106 to syngas. As will be described in further detail, the biomass enters the system 100 upstream of the gasification reactor 102 and is processed into feed 104a prior to being fed to the gasification reactor. Feed 104a is in a form suitable for efficient processing in the gasification reactor 102. The oxygen feed 106 is supplied by an air-separation unit 112 and an electrolysis unit 114. The air-separation unit 112 and electrolysis unit 114 may be operated simultaneously so that oxygen is supplied to the gasification reactor 102 by the air-separation unit 112 and the electrolysis unit 114. Alternatively, only one of the air-separation unit 112 and electrolysis unit 114 may be operated so that oxygen from only one of the air-separation unit 112 and electrolysis unit 114 is supplied to the gasification reactor 102. Prior to being fed to the gasification reactor, oxygen feed 106 is passed through compressor 117. Within the gasification reactor 102 biomass feed 104a is gasified in the presence of the oxygen feed 106 to produce syngas (a mixture of $H_2$, CO and $CO_2$). The raw syngas exits the gasifier 102 as syngas stream 108a. Slag is a waste product of the gasification process and exits the gasification reactor 102 as slag stream 110.

When operating, the air-separation unit 112 separates oxygen from atmospheric air 113 and supplies oxygen to the gasification reactor 102 as at least a portion of the oxygen feed 106. The air-separation unit 112 is preferably powered by green electricity obtained, for example, from wind power, solar power, geothermal power or hydropower. The maximum output of a single air-separation unit is typically about 60-65 t/h of oxygen. The system 100 may comprise multiple air-separation units 112 so that if the oxygen demand of the gasification reactor exceeds the maximum output of a single air-separation unit, further air-separation units may be operated.

The electrolysis unit 114 comprises multiple alkaline electrolysis cells arranged in stacks (not shown). The electrolysis unit 114 receives a water feed 115 and the water is split into hydrogen and oxygen by the electrolysis cells. When unit 114 is operating the oxygen is supplied to the gasification reactor 102 as at least portion of the oxygen feed 106. The hydrogen is fed as a hydrogen stream 118 to a methanol synthesis reactor 116 downstream of the gasification reactor 102. The hydrogen leaving the electrolysis unit has a pressure of about 1.2 bar and is compressed to about 72 bar by compressor 120 before being fed to the methanol synthesis reactor 116. The electrolysis unit 114 is preferably supplied by green electricity (such as from wind power, solar power, geothermal power or hydropower) and therefore the hydrogen produced may be referred to as 'green hydrogen'. Each electrolysis cell has a power output of about 1-3 MW and the cells are coupled together in stacks of up to 100MW. If the oxygen and/or hydrogen demand of system 100 exceeds the maximum output of the electrolysis unit 114, further electrolysis units may be added and operated.

Raw syngas stream 108a is passed through a scrubber unit 122 to remove contaminants, such as HCl, halides, fine particles of dust and liquid aerosols. Syngas condensate and boiler feed water may be used in the scrubbing phase as a washing liquid. To promote the HCl and other halide absorption, NaOH solution can be used in the process.

A cleaned syngas stream 108b produced by the scrubber unit 122 is then passed to a water-gas shift reactor 124. The water-gas shift reactor 124 converts the syngas stream 108b to a hydrogen-rich syngas 108c by increasing the hydrogen content of the syngas. Carbon monoxide of the syngas 108b and water (steam) are converted to carbon dioxide and hydrogen in a water-gas shift process, thus increasing the hydrogen content of the syngas. This improves the stoichiometric number of the syngas to that needed for efficient methanol synthesis. Preferably, the stoichiometric number of the syngas once it has been processed in the water-gas shift reactor 124 is about 2.0-2.1.

Hydrogen-rich syngas stream 108c exits the water-gas shift reactor 124 and is passed to an acid-gas removal unit 126 located downstream of the water-gas shift reactor 124. The acid-gas removal unit 126 removes acidic gases such as carbon dioxide and gases comprising sulfur e.g. $H_2S$. Removal of carbon dioxide from the syngas 108c improves the stoichiometric number of the syngas 108c. Sulfur gases are removed from the syngas gas 108c because they can poison the catalyst used in the methanol synthesis reactor 116. A portion 127 of the carbon dioxide can be recycled to upstream of the gasification reactor 102 to be used for purging, sluicing and feeding biomass 108a to the gasification reactor 102. Remaining carbon dioxide is collected and sequestered or released to the atmosphere as biogenic carbon dioxide.

The acid-gas removal unit 126 comprises an absorber/desorber column (not shown) that uses an aqueous amine solution to remove the acidic components from the syngas according to methods known in the art. The acid-gas removal unit 126 also comprises two absorber columns (not shown) arranged in series and each comprising sulphur guard bed absorbers. The sulphur guard bed absorbers remove the sulfur gases remaining in the syngas after being passed through the absorber/desorber column to achieve a high purity syngas. The high purity syngas exists the acid-gas removal unit 126 as syngas stream 108d.

The high purity syngas 108d produced by the acid-gas removal unit 126 is converted to methanol in methanol synthesis reactor 116. The pressure of the syngas stream 108d is increased to about 70 bar in compressor 128 before being fed to the methanol synthesis reactor 116. Hydrogen stream 118 is also supplied to the methanol synthesis reactor 116 to improve the stoichiometric ratio of the syngas 108d, as is described herein. As shown in Figure 1, the syngas feed 108d and hydrogen feed 118 are delivered to the methanol synthesis reactor 116 as a combined stream 108d. However, it will be understood that in embodiments of the invention the syngas feed 108d may be combined with the hydrogen feed 118 within the methanol synthesis reactor 116.

The methanol synthesis process carried out in the methanol synthesis reactor 116 employs a $Cu/ZnO/Al_2O_3$ catalyst for converting syngas to methanol. It will be understood that in embodiments, any suitable catalyst system for converting syngas to methanol may be used. Suitable reaction conditions are a temperature of about 210-250 °C and a pressure of about 70 bar.

The raw methanol produced by the methanol synthesis reactor 116 is discharged from the methanol synthesis reactor 116 as raw methanol stream 130. The raw methanol stream 130 comprises impurities such as water, dissolved gases and byproducts such as dimethyl ether, methyl formate and higher alcohols e.g. ethanol, propanol and butanol. The raw methanol stream 130 is passed to distillation unit132 to remove the impurities by distillation. Methanol distillate is produced and removed from the distillation column 130 as methanol distillate stream 134. Light and heavy impurity streams (not shown) also leave distillation unit 132.

As shown in Figure 1, system 100 further comprises a bypass conduit 136 which diverts at least a portion of syngas (i.e. syngas upstream of the water-gas shift reactor 124) to a location downstream of the water-gas shift reactor 124 and without passing through the water-gas shift reactor 124. As shown in Figure 1, the bypass conduit 138 directs syngas to a location immediately downstream of the water-gas shift reactor and upstream of the acid gas removal unit 126 (pathway labelled 136a). The bypass conduit 136 also diverts syngas which is mixed with hydrogen stream 118 prior to the combined stream entering the methanol synthesis reactor 116 (pathway labelled 136b). The bypass conduit 136 comprises a valve (not shown) for controlling the flow of syngas 108b through pathway 136a and 136b. Through use of the valve, syngas 108b can be directed entirely though pathway 136a, entirely through pathway 136b or through both pathways. It will be understood that alternative configurations are possible provided that the bypass conduit 136 does not pass through the water-gas shift reactor 124. For example, in some embodiments bypass conduit 136 may direct syngas immediately downstream of the water-gas shift reactor and upstream of the acid gas removal unit 126 only (pathway labelled 136a). In alternative embodiments, the bypass conduit 136 diverts syngas 108b to be mixed with hydrogen stream 118 only and prior to the combined stream entering the methanol synthesis rector 116 (pathway 108b).

The system 100 further comprises a bypass control system for controlling the flow of syngas to the water-gas shift reactor and the flow of syngas through the bypass conduit. In the exemplary embodiment shown in Figure 1, the bypass control system comprises a control valve 138 for controlling the flow of syngas stream through the bypass conduit 136 In Figure 1, the control valve 138 is represented as a three-way valve for controlling the flow of syngas stream 108b to the water-gas shift reactor 124 and the flow of syngas through the bypass conduit 136. The control valve 138 is operated to control the amount of syngas bypassing the water-gas shift reactor 124 and in dependence on the amount of hydrogen 118 that is fed to the methanol synthesis reactor 116, as described herein. In some embodiments, alternative valve arrangements may be used, provided they allow control of the flow of syngas through the bypass conduit 136. For example, the system may comprise a first two-way valve located in bypass conduit 138 for controlling the flow of syngas 108b through the bypass conduit 136, and optionally a second two-way valve at the inlet of the water-gas shift reactor 124 for controlling flow of syngas 108b into the water-gas shift reactor.

The system 100 may be operated to convert biomass to methanol in a gasification-only mode, full-carbon capture mode, or an intermediate mode. In the gasification-only mode, the electrolysis unit 114 is not operated and all hydrogen used in the process is provided by the gasification reactor 102 and the water-gas shift unit 124. The air-separation unit 112 supplies 106 oxygen to the gasification reactor. The raw syngas 108a produced by the gasification reactor 102 comprises a mixture or $H_2$, CO and $CO_2$, and has a stoichiometric number (defined by Eq. 3) outside of the desired stoichiometric number for methanol synthesis of about 2.0-2.1. To adjust the stoichiometric number of the syngas, syngas stream 108b is passed through the water-gas shift unit 124 and undergoes a water-gas shift process (Eq. 4) thereby converting steam and carbon monoxide to carbon dioxide and hydrogen. As a consequence of increasing the hydrogen content of the syngas 108c, excess carbon dioxide is produced which is removed by acid gas removal unit 126. Syngas stream 108d exiting the acid-gas removal unit 126 has a stoichiometric number of about 2.0-2.1. In the gasification-only mode, no syngas is passed through bypass conduit 136, thus control valve 138 is positioned so that all syngas stream 108b is directed through water-gas shift reactor 124.

In a full-carbon recovery mode, the electrolysis unit 114 is operated and hydrogen stream 118a is supplied to methanol reactor 116. In full-carbon recovery mode, the electrolysis unit 114 meets the hydrogen demand of the system. The syngas stream is diverted around the water-gas shift unit 124 via bypass conduit 136. In the full-carbon recovery mode, the control valve 138 is positioned so that all syngas stream is directed through the bypass conduit 138 and none passes through the water-gas shift reactor 124. Because the water-gas shift unit 124 is bypassed, carbon monoxide is not converted to carbon dioxide, minimising carbon that is removed from the system as carbon dioxide byproduct and maximising the amount of carbon converted to methanol. In the full-carbon recovery mode, the air-separation unit 112 is not operated as the electrolysis unit 114 meets the oxygen demand of the gasification reactor.

In an intermediate mode, the electrolysis unit 114 is operated below maximum capacity. The electrolysis unit 114 provides a portion of the hydrogen needed to adjust the stoichiometric number of the syngas to be within about 2.0-2.1, however, further hydrogen must be generated by the water-gas shift unit 124 to meet the hydrogen demand of the system. In the intermediate mode, the control valve 138 is positioned so that a portion of syngas stream is directed through bypass conduit 136, thus bypassing water-gas shift unit 124, and the remainder of syngas stream 108b is passed through water-gas shift unit 124. It will be understood that the portion of syngas stream 108b directed through bypass conduit 136 is adjusted in dependence on the hydrogen output of electrolysis unit 114. As more hydrogen is produced by electrolysis unit 118, the control valve 138 is adjusted so that more syngas stream 108b passes through bypass conduit 136.

System 100 comprises a controller 140 for monitoring the stoichiometric number of the combined stream 108e entering the methanol synthesis reactor 116. The stoichiometric number of the combined stream 108e entering the methanol synthesis reactor 116 is determined, for example by determining the composition of the combined stream using thermal conductivity or gas chromatography, and that data is provided as an input 142 to controller 140. The controller 140 compares the input 142 to a reference stoichiometric range (e.g. 2.0-2.1). If the input 142 lies outside of the reference stoichiometric range, then the controller 142 initiates adjustment 144 of the position of the control valve 138. If more syngas is required to pass through the water-gas shift unit 124 to reach a stoichiometric value within the range of 2.0-2.1, then the position of the control valve 138 is adjusted so that the flow of syngas to the water-gas shift reactor is increased and the flow of syngas 108b through the bypass conduit 138 is decreased. If less syngas is required to pass through the water-gas shift unit 124 to reach a stoichiometric value within the range of 2.0-2.1, then the control valve 138 is adjusted so that the flow of syngas to the water-gas shift reactor is decreased and the flow of syngas 108b through the bypass conduit 138 is increased. Additionally or alternatively, the controller 140 may monitor the stoichiometric number of the hydrogen-rich syngas stream when the stoichiometric number of the hydrogen-rich syngas stream is determined.

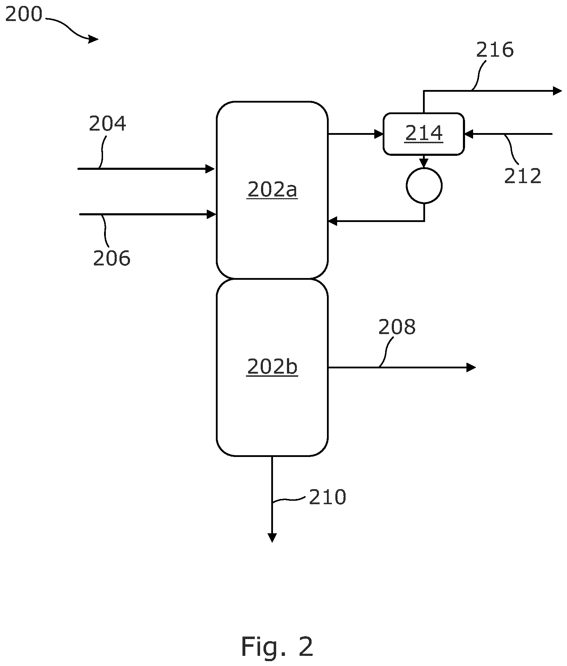

The gasification reactor 102 of Figure 1 may be in the form of an entrained flow gasifier or a fluidised bed gasifier. An example of an entrained flow gasifier is shown in Figure 2. The entrained flow gasifier 200 of Figure 2 is divided into two sections 202a and 202b. The gasification reactor is located in the upper section 202a and a quenching chamber is located in the lower section 202b. Biomass feed 104a of Figure 1 enters the gasification reactor 200 of Figure 2 as stream 204. The oxygen feed 106 of Figure 1 enters the gasification reactor 200 of Figure 2 as stream 206. Within the gasification reactor the biomass and oxygen stream are converted to a raw syngas. The gasification reactor operates at about 40-45 bar and a temperature of about 210-220 °C and produces about 330 - 340 t/h of raw syngas. Raw syngas and fly ash produced in the gasification reactor leave the gasification reactor of upper section 202a through an opening (not shown) at the bottom of the upper section 202a and enters a quenching chamber (not shown) of lower section 202b. Quench water is sprayed at different levels within the quenching chamber to saturate the syngas and solidify the fly ash as slag. The syngas and slag are separated in lower section 202b. The saturated raw syngas 208 leaves the pressure vessel with a temperature of about 220 °C and forms the syngas stream 108a of Figure 1. Slag leaves the entrained flow gasifier 200 as slag stream 210 which is collected for further processing as described herein. The temperature of the gasification reactor is maintained by one or more heat exchange systems (not shown) and water is used as the heat transfer medium. A boiler feed water stream 212 is fed via steam drum 214 to the gasification reactor 200 in upper section 202a for use as the cooling medium. The water is returned to the steam drum, generating a high pressure steam stream 216 for use in the central heat recovery system, as described herein.

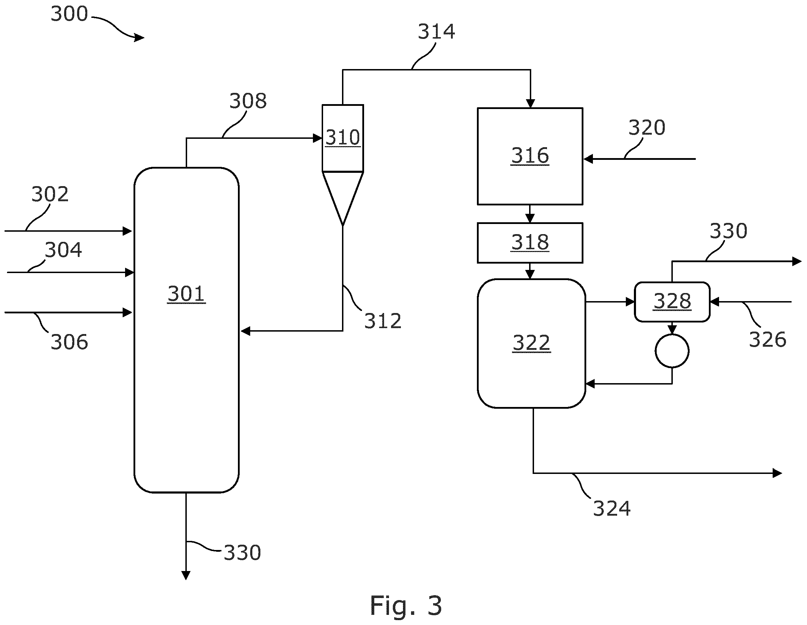

Additionally or alternatively, the gasification reactor 102 may be in the form of a fluidised bed gasifier. An example of a fluidised bed gasifier is shown in Figure 3. Fluidised bed reactor 300 operates at about 9-12 bar, a temperature of about 350-360 °C and produces about 91 t/h of raw syngas. The fluidised bed reactor 300 receives biomass feed 104a of Figure 1 as stream 302 and the oxygen feed 106 of Figure 1 enters the fluidised bed reactor 300 as stream 304. High-pressure superheated steam is supplied to the fluidised bed reactor as superheated steam stream 306. The biomass stream 302 is gasified in the fluidised bed reactor chamber 301 in the presence of oxygen supplied by oxygen stream 304 and high-pressure superheated steam supplied by superheated steam stream 306. Slag exits the fluidised bed reactor chamber 301 as slag stream 330. The raw syngas produced in the gasification reaction exits the fluidised bed gasifier chamber 301 as raw syngas stream 308. Entrained particles of fluidised bed material are substantially removed from the raw syngas stream 308 in cyclone 310 and returned to the fluidised bed gasifier 300 via cyclone return conduit 312. The syngas stream 314 exiting the cyclone 310, including particulate matter and char, enters a hot oxygen burner 316. The hot oxygen burner 316 provides heat for the partial oxidation (POX) reactor vessel 318 to reach tar destruction temperature. The desired temperature of destruction for tars is achieved by injecting oxygen feed 320 and a portion of syngas stream 314 as a fuel into the hot oxygen burner 316. The POX reactor vessel 318 converts unconverted carbon, tar components and other unsaturated hydrocarbon compounds into hydrogen and carbon monoxide. Syngas exits the POX reactor vessel 318 and is cooled in a syngas cooler system 322 to produce cooled syngas stream 324. The syngas cooler 322 is cooled by at least one heat exchanger (not shown) using water 326 as the heat transfer medium. A heat recovery system comprising stream drum 328 recovers heat from the water of the heat exchanger to generate high pressure stream 330. Molten slag present in the syngas stream 314 is solidified within the syngas cooler 322. The solidified and cooled slag disengages from the syngas stream and drops by gravity through the syngas cooler 322 into a slag removal system (not shown).

Referring back to Figure 1, raw biomass is processed prior to being delivered to the gasification reactor 102 as biomass feed 104a. Processing of the raw biomass improves the efficiency of the gasification process. The system 100 comprises a drying unit 141 for removing water from the raw biomass feed 104b in which hot air is passed over the raw biomass feed 104b to remove a majority of the water content. Typically the raw biomass 104b has a water content in the region of 40-50 wt%. A dried biomass feed 104c is discharged from the drying unit 140 and is passed to a torrefaction unit 143. Within the torrefaction unit 143, the dried biomass feed 104c is exposed to temperatures of about 250-300 °C in a torrefaction process. The torrefaction process partially devolatizes the biomass and weakens the fibrous structure of the biomass to improve the grindability of the biomass. Volatiles 156 produced in the torrefaction process are combusted in combustor 154. A heat exchanger 158 of a heat exchange system transfers heat from the exhaust gas of the combustor 154 to an air stream to form a hot air stream 160 which is used in drying unit 141 to dry the raw biomass as disclosed herein.

The torrefied biomass is discharged from the torrefaction unit 143 as a torrefied biomass feed 104d which is then pelletized in pelletizer 145 and ground into a fine powder in grinder 147 before being transferred by pneumatic conveying from feed unit 150 to the gasification reactor 102. The feed unit 150 comprises (not shown) a silo for collecting torrefied biomass. The feed unit 150 further comprises (not shown) pressure and surge hoppers for receiving torrefied biomass from the silo, pressurising the biomass to a pressure suitable for delivery to the gasification reactor 102, and delivering pressurised biomass to the gasification reactor 102 at a regulated rate. The torrefied biomass is pressurised in the pressure and surge hoppers by carbon dioxide stream 127 discharged from acid-gas removal unit 126. Prior to being fed to the pressure and surge hoppers, carbon dioxide stream 127 is passed through compressor 129.

A portion of the slag 110 discharged from the gasification reactor 102 is recycled to upstream of the gasification reactor 102 as recycle slag stream 152 and is combined with the torrefied biomass. The combined recycled slag and torrefied biomass stream is supplied to the gasification reactor. As an example, 3-3.5 t/h of slag per hour are combined with the torrefied biomass stream upstream of the gasification reactor.

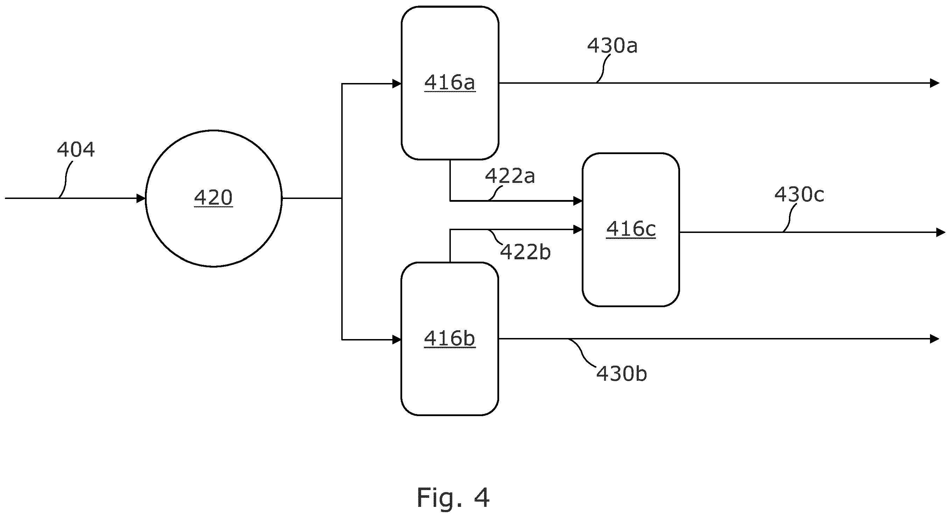

The system 100 may comprise more than one methanol synthesis reactor. An example of an arrangement of three methanol synthesis reactors is shown in Figure 4. The combined stream 108e and optionally the hydrogen stream 118 of Figure 1, are delivered to methanol synthesis reactors 416a, 416b as syngas stream 404 via compressor 420. A first methanol synthesis reactor 416a and second methanol synthesis reactor 416b are arranged in parallel, each reactor 416a, 416b receiving a portion of syngas stream 404. Crude methanol streams 430a, 430b are discharged from the methanol synthesis reactors 416a, 416b and are purified in one or more distillation columns as disclosed herein. Unreacted syngas, if present, is separated from the syngas and passed to a third methanol synthesis reactor 416c as unreacted syngas streams 422a, 422b for conversion to methanol. Crude methanol stream 430c is discharged from the methanol synthesis reactor 416c and purified in one or more distillation columns as disclosed herein. As an example, each methanol synthesis reactor 416a, 416b, 416c has a maximum output of about 75 t/h of methanol.

Although the third methanol synthesis reactor 416c is present, the third methanol synthesis reactor 416b is not operated in gasification-only mode. The first and second methanol synthesis reactors 416a, 416b have a capacity such that in gasification-only mode, the first and second methanol synthesis reactors 416a, 416b can fully convert syngas stream 404. In full-carbon recovery mode, the syngas feed rate to the methanol synthesis reactor exceeds the maximum capacity of the first and second methanol synthesis reactors 416a, 416b. In full-carbon recovery mode, the third methanol synthesis reactor 416c is operated at maximum capacity and converts unreacted syngas discharged from the first and second methanol synthesis reactors 416a, 416b to methanol. Thus, the system provides flexibility to switch between gasification-only and full-carbon capture mode easily and quickly, without modification of the system. It will be understood that in an intermediate mode, the third methanol synthesis reactor 416c is operational but not at full capacity.

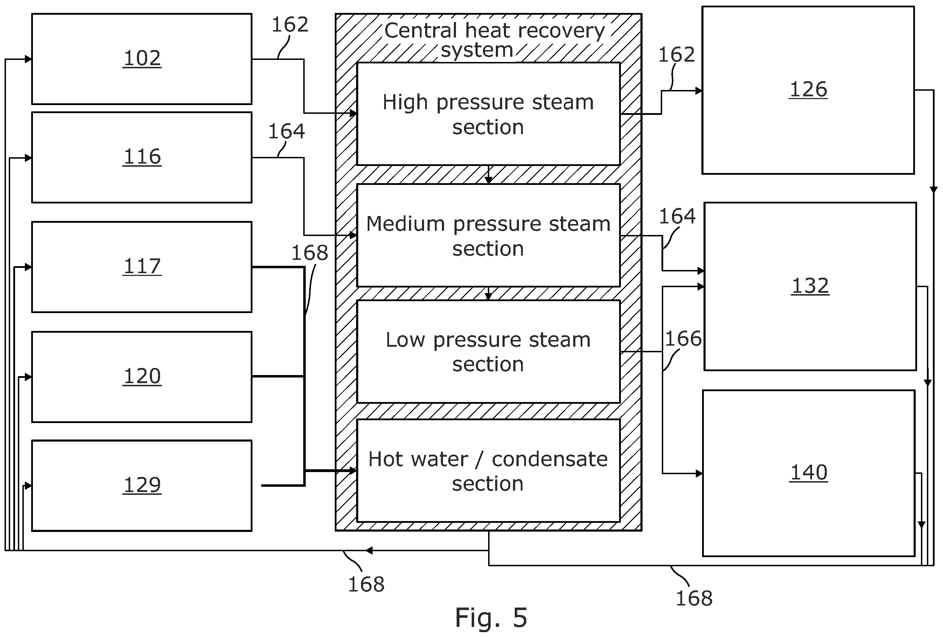

Waste heat from the system 100 is recovered to provide heat and/or useful work elsewhere in the system 100. Figure 5 is a flow diagram of heat transfer within system 100. Units of system 100 that generate heat include gasification reactor 102, methanol synthesis reactor 116, oxygen compressor 117, hydrogen compressor 120, and carbon dioxide compressor 129. Each of those units comprises one or more heat exchange systems (not shown) for controlling the temperature of the unit and producing steam using recovered heat and/or hot water or condensate. Optionally, the water-gas shift reactor (not shown) may also be integrated with the central heat recovery system. The water-gas shift reactor may comprise a heat exchange system for cooling the hydrogen-rich syngas produced by the water-gas shift reactor. Said heat exchange system may supply heat, e.g. in the form of steam, to the heat recovery system.

Steam and hot water/condensate generated in those units are fed into a central heat recovery system. High pressure steam 162 having pressure of about 50 bar and a temperature of about 265 °C is formed by the heat exchange system of the gasification reactor 102. Medium pressure steam 164 having pressure of about 30 bar and a temperature of about 234 °C is formed by the heat exchange system of the methanol synthesis reactor 116. The heat exchange systems of the oxygen compressor 117, hydrogen compressor 120, and carbon dioxide compressor 129 produce hot water and/or condensate 168. Hot water/condensate 168 produced by the acid-gas removal unit 126, methanol distillation column 132 and drying unit 140 is fed to the central heat recovery system The central heat recovery system comprises cascading stages to expand the steam to the desired pressure. For example, high pressure steam 162 is expanded to medium pressure steam 164 and medium pressure steam is expanded to low pressure steam 166 having a pressure of about 7 bar and a temperature of about 168 °C.

High pressure steam 162 is supplied to the acid-gas removal unit 126. Medium pressure steam 164 is supplied to methanol distillation column 132 to provide heat for the distillation of crude methanol as described herein. Low pressure steam 166 is also supplied to methanol distillation column 132. For example, the medium pressure steam 164 and low pressure steam 166 may be supplied to the methanol distillation column 132 to provide heat for startup of the column. Low pressure steam 166 is also supplied to drying unit 140 to provide heat for drying as described herein. Hot water/condensate 168 is recycled back to the heat exchange systems of each of the gasification reactor 102, methanol synthesis reactor 116, oxygen compressor 117, hydrogen compressor 120, and carbon dioxide compressor 129.

Steam may also be fed to steam turbines for driving various compressors used in the system 100. For example, the high pressure steam 162 or medium pressure steam 164 may be expanded in one or more steam turbines to produce shaft power for mechanical work. For example, high and/or medium pressure steam may be provided to oxygen compressor 117, hydrogen compressor 120, and carbon dioxide compressor 129.

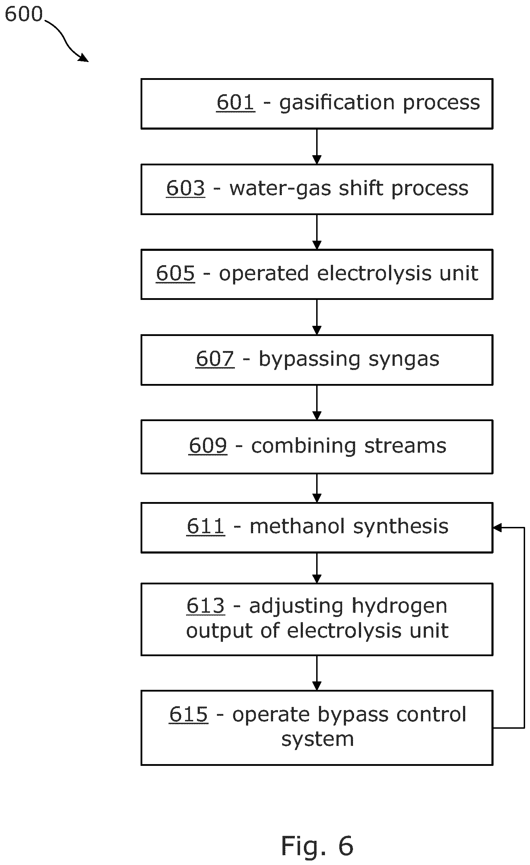

Figure 6 is a flow diagram of a method 600 according to the present invention in which in a first step 601 a processed biomass feed and an oxygen feed are converted to a syngas stream in the gasification reactor. In a second step 603, at least a portion of the syngas stream is fed to the water-gas shift reactor to produce a hydrogen-rich syngas stream. In step 605, the electrolysis unit is operated to produce a hydrogen stream. In step 607, a portion of the syngas stream is passed through the bypass conduit and without passing through the water-gas shift reactor. In step 609, the hydrogen-rich syngas stream, syngas from the bypass conduit and the hydrogen stream are combined to form a combined stream.

In step 611, the combined stream is converted to methanol in the methanol synthesis reactor. In step 613, the hydrogen stream output of the electrolysis unit is adjusted. In step 615, the bypass control system is operated to maintain the stoichiometric number of the combined stream within a predetermined range, wherein the stoichiometric number (SN) is defined as:

$$SN = ([H_2] - [CO_2])/([CO] + [CO_2])$$

and $[H_2]$ is the hydrogen concentration of the combined stream, $[CO_2]$ is the carbon dioxide concentration of the combined stream and $[CO]$ is the carbon monoxide concentration of the combined stream.

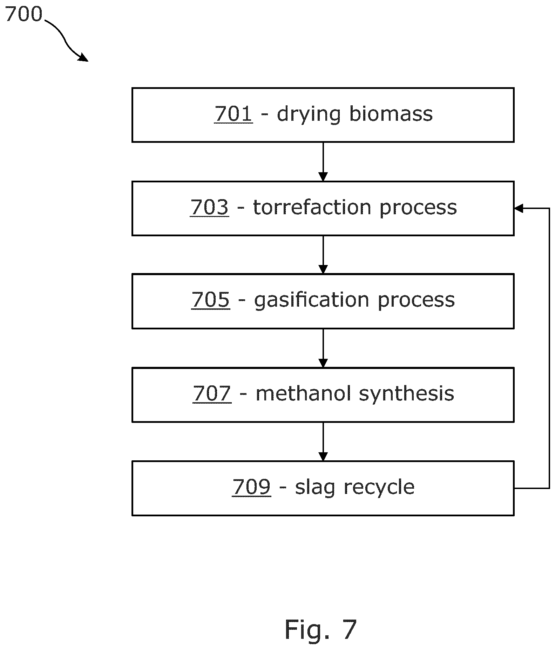

Figure 7 shows a method 700 of the present invention in which raw biomass is dried in step 701 to produce a dried biomass. In step 703, the dried biomass is converted to a torrefied biomass feed in a torrefaction process in a torrefaction unit. In step 705, the torrefied biomass is converted to a syngas feed in a gasification process in a gasification reactor. In step 707, the syngas feed is converted into methanol in a methanol synthesis reactor. Slag is produced as a waste product of the gasification process and the method 700 further comprises recycling a first portion of slag in step 709 to be mixed with at least one of the raw biomass feed, dried biomass feed and torrefied biomass feed upstream of the gasification reactor.

Whilst the present invention has been described and illustrated with reference to particular embodiments, it will be appreciated by those of ordinary skill in the art that the invention lends itself to many different variations not specifically illustrated herein. By way of example only, certain possible variations will now be described. The gasification reactor may be a fluidised bed gasifier, an entrained flow gasifier or a combination of both. The system may comprise multiple of each component to increase the capacity of the system. For example, the system may comprise multiple gasification reactors, methanol synthesis reactor, methanol distillation columns, electrolysis units, air-separation units and water-gas shift units. It will be understood that the system further comprises components not specifically mentioned but used in the art for example compressors, heat exchange systems, valves, conduits, pressure relief valves and power supplies. Although steps of figures 6 and 7 are shown sequentially, it will be understood that the steps may be performed simultaneously.

Where in the foregoing description, integers or elements are mentioned which have known, obvious or foreseeable equivalents, then such equivalents are herein incorporated as if individually set forth. Reference should be made to the claims for determining the true scope of the present invention, which should be construed so as to encompass any such equivalents. It will also be appreciated by the reader that integers or features of the invention that are described as preferable, advantageous, convenient or the like are optional and do not limit the scope of the independent claims. Moreover, it is to be understood that such optional integers or features, whilst of possible benefit in some embodiments of the invention, may not be desirable, and may therefore be absent, in other embodiments.

Embodiments of the invention will now by described with reference to the following numbered clauses.

Clause 1. A system for converting biomass to methanol, the system comprising:

a gasification reactor comprising a biomass feed inlet and a syngas outlet;
a water-gas shift reactor located downstream of the gasification reactor and comprising a syngas stream inlet and hydrogen-rich syngas stream outlet;
a first methanol synthesis reactor located downstream of the water-gas shift reactor;
a bypass conduit fluidly connecting the syngas outlet of the gasification reactor with the first methanol synthesis reactor, wherein the bypass conduit does not pass through the water-gas shift reactor;
a bypass control system for controlling the flow of syngas to the water-gas shift reactor and the flow of syngas through the bypass conduit;
an air-separation unit comprising an air inlet and an oxygen outlet; and,
an electrolysis unit comprising a water inlet, an oxygen outlet and a hydrogen outlet;

wherein the gasification reactor is arranged to receive:

(i) oxygen from the oxygen outlet of the air-separation unit; and/or,
(ii) oxygen from the oxygen outlet of the electrolysis unit;
and the first methanol synthesis reactor is arranged to receive:
(iii) hydrogen-rich syngas from the hydrogen-rich syngas stream outlet of water-gas shift reactor; and/or,
(iv) syngas from the bypass conduit and hydrogen from the hydrogen outlet of the electrolysis unit.

Clause 2. The system of clause 1, wherein the gasification reactor is an entrained flow gasifier, optionally wherein the entrained flow gasifier has a maximum syngas stream output of at least 300 t/h.

Clause 3. The system of clause 1 or clause 2, wherein the gasification reactor is a fluidized bed gasifier, optionally wherein the fluidized bed gasifier has a maximum syngas stream output of at least 80 t/h.

Clause 4. The system of any preceding clause, wherein the system comprises multiple gasification reactors; optionally, wherein the system comprises a combination of at least one entrained flow gasifier and at least one fluidized bed gasifier.

Clause 5. The system of any preceding clause, further comprising a first distillation column for producing a methanol distillate, wherein the first distillation column comprises an inlet for receiving methanol effluent from the first methanol synthesis reactor and an outlet for discharging a methanol distillate having a higher content of methanol by weight % than the methanol effluent;
optionally, wherein the methanol distillate has a methanol content of at least 99.85 wt% methanol effluent.

Clause 6. The system of any preceding clause, further comprising a second and a third methanol synthesis reactor for converting syngas to methanol;

wherein the first methanol synthesis reactor and the second methanol synthesis reactor are arranged in parallel so that both of the first and second methanol synthesis reactors are arranged to receive:

(iii) the hydrogen-rich syngas from the hydrogen-rich syngas stream outlet of water-gas shift reactor; and/or,

(iv) the syngas from the bypass conduit and hydrogen from the hydrogen outlet of the electrolysis unit; and,

wherein each of the first and second methanol synthesis reactors comprise:

an outlet for discharging methanol; and,
an outlet for discharging unreacted syngas;

wherein the third methanol synthesis reactor is arranged in series with the first and second methanol synthesis reactor, and is arranged to receive methanol effluent from the outlet of the first methanol synthesis reactor and/or the outlet of the second methanol synthesis reactor; and, an outlet for discharging a methanol effluent.

Clause 7. The system of clause 6, further comprising a second and third methanol distillation column for producing a methanol distillate;

wherein the inlet of the first methanol distillation column is for receiving methanol effluent from the first methanol synthesis reactor;
the second methanol distillation column has an inlet for receiving methanol effluent from the second methanol synthesis reactor; and,
the third methanol distillation column has an inlet for receiving methanol effluent from the third methanol synthesis reactor;
wherein each of the first, second and third methanol distillation columns comprise an outlet for discharging a methanol distillate having a higher content of methanol by weight % than the methanol effluent;
optionally, wherein the methanol distillate of each methanol distillation column has a methanol content of at least 99.85 wt% methanol effluent.

Clause 8. The system according to any preceding clause, further comprising an acid gas removal unit for removing acid gases from syngas, wherein the acid gas removal unit is located downstream of the water-gas shift reactor and upstream of the first methanol synthesis reactor,

wherein the acid gas removal unit has an inlet for receiving hydrogen-rich syngas from the water-gas shift reactor, and optionally syngas from the bypass conduit; an absorber/desorber system for acid gas recovery; an outlet for discharging recovered acid gas; and an outlet for cleaned syngas;
optionally, wherein the recovered acid gas comprises carbon dioxide and the absorber/desorber system comprises an amine-absorber/desorber system.

Clause 9. A method for converting biomass to methanol in a system comprising:

a gasification reactor;
a water-gas shift reactor located downstream of the gasification reactor;
a first methanol synthesis reactor located downstream of the water-gas shift reactor;
a bypass conduit for diverting syngas from upstream of the water-gas shift reactor to downstream of the water-gas shift reactor and without passing through the water-gas shift reactor;
a bypass control system for controlling the flow of syngas to the water-gas shift reactor and the flow of syngas through the bypass conduit;
an air-separation unit for supplying oxygen to the gasification reactor; and,
an electrolysis unit for supplying oxygen to the gasification reactor and
hydrogen to a first methanol synthesis reactor;
wherein the method comprises: converting a biomass feed and an oxygen feed to a syngas stream in the gasification reactor;
feeding at least a portion of the syngas stream to the water-gas shift reactor to produce a hydrogen-rich syngas stream;
operating the electrolysis unit to produce a hydrogen stream;
feeding at least a portion of the syngas stream through the bypass conduit and without passing through the water-gas shift reactor;
combining the hydrogen-rich syngas stream, syngas from the bypass conduit and the hydrogen stream to form a combined stream; and,
converting the combined stream to methanol in the first methanol synthesis reactor;
wherein the method further comprises:

adjusting the hydrogen stream output of the electrolysis unit; and,
operating the bypass control system to maintain the stoichiometric number of the combined stream within a predetermined range, wherein the stoichiometric number (SN) is defined as:

$$SN = ([H_2] - [CO_2])/([CO] + [CO_2])$$

and $[H_2]$ is the hydrogen concentration of the combined stream, $[CO_2]$ is the carbon dioxide concentration of the combined stream and $[CO]$ is the carbon monoxide concentration of the combined stream.

Clause 10. The method of clause 9, wherein the predetermined range is 2.0 to 2.1.

Clause 11. The method of clause 9 or clause 10, further comprising operating the bypass control system so that all of the syngas stream passes through the water-gas shift reactor to form the hydrogen-rich syngas, and converting the hydrogen-rich syngas stream to methanol in the first methanol synthesis reactor.

Clause 12. The method of clause 9 or clause 10, further comprising operating the bypass control system so that all of the syngas stream passes through the bypass conduit, and converting the syngas from the bypass conduit and the hydrogen stream from the electrolysis unit to methanol in the first methanol synthesis reactor.

Clause 13. The method of any preceding clause, wherein the air-separation unit supplies at least a portion of the oxygen feed of the gasification reactor and the electrolysis unit also supplies at least a portion of the oxygen feed of the gasification reactor, and the method further comprises:
adjusting the flow of oxygen from the air-separation unit to the gasification reactor and the flow of oxygen from the electrolysis unit to the gasification reactor in response to the adjustment of the hydrogen stream output of the electrolysis unit.

Clause 14. The method of clause 13, wherein when the hydrogen stream output of the electrolysis unit is increased, the method further comprises increasing the flow of oxygen from the electrolysis unit to the gasification reactor and decreasing the flow of oxygen from the air-separation unit to the gasification reactor; or,
wherein when the hydrogen stream output of the electrolysis unit is decreased, the method further comprises decreasing the flow of oxygen from the electrolysis unit to the gasification reactor and increasing the flow of oxygen from the air-separation unit to the gasification reactor.

Clause 15. The method of any one of clause 9 to 14, wherein the system comprises a sample outlet for venting a sample of the combined stream and a detector for determining the stoichiometric number of the sample of the combined stream; and,
the bypass control system further comprises a controller and at least one control valve and operating the bypass control system to maintain the stoichiometric number of the combined stream within a predetermined range comprises:

determining the stoichiometric number of a sample of the combined stream vented from the sample outlet using the detector;
providing the stoichiometric number of the combined stream as a data input into the controller;
comparing the stoichiometric number of the combined syngas to the predetermined range stored on the controller as a reference stoichiometric range and,
when the stoichiometric number of the combined stream is outside of the reference stoichiometric range, adjusting the at least one control valve to adjust the flow of the syngas to the water-gas shift reactor and/or the flow of syngas through the bypass conduit.

Clause 16. The method of any one of clauses 9 to 15, wherein the system is according to any one of clauses 1 to 8.

Clause 17. A system for converting biomass to methanol, the system comprising:

a drying unit for drying raw biomass to produce a dried biomass feed;
a gasification reactor for converting the dried biomass feed and an oxygen feed to syngas;
a water-gas shift reactor for increasing the hydrogen content of the syngas to form a hydrogen-rich syngas stream;

EP 4 763 828 A1

an acid gas removal unit for removing acidic gases from the hydrogen-rich syngas stream;
a first methanol synthesis reactor for converting the hydrogen-rich syngas to crude methanol; and,
a methanol distillation column for purifying the crude methanol to produce a methanol distillate;
wherein each of the gasification reactor and first methanol synthesis reactor comprise a heat exchange system for controlling the temperature of the gasification reactor and/or methanol synthesis reactor and producing steam using recovered heat; and,
each of the drying unit, acid-gas removal unit and methanol distillation column comprise a heat exchange system for transferring heat from the steam to the drying unit, acid-gas removal unit or methanol distillation column;
wherein the system further comprises a central heat recovery system for receiving the steam from the heat exchange systems of the gasification reactor and first methanol synthesis reactor and delivering the steam to the heat exchange system of each of the drying unit, acid-gas removal unit and methanol distillation column.

Clause 18. The system of clause 17, wherein the heat exchange system of the gasification reactor produces a high pressure steam having a pressure of at least 45 bar and a temperature of at least 255 °C; and,
the central heat recovery system comprises a high pressure steam section for receiving the high pressure steam and delivering the high pressure steam to the heat exchange system of the acid-gas removal unit.

Clause 19. The system of clause 17 or clause 18, wherein the heat exchange system of the first methanol synthesis reactor produces a medium pressure steam having a pressure of greater than or equal to 25 bar and less than 45 bar and a temperature of greater than or equal to 220 °C and less than 255 °C; and,
the central heat recovery system comprises a medium pressure steam section for receiving the medium pressure steam and delivering the medium pressure steam to the heat exchange system of the methanol distillation column.

Clause 20. The system of clauses 18 and 19, wherein the central heat recovery system comprises a cascading stage between the high pressure steam section and medium pressure steam section for expanding a portion of the high pressure steam of the high pressure section to a medium pressure steam;
optionally, wherein the central heat recovery system comprises a cascading stage between the medium pressure steam section and a low pressure steam section for expanding a portion of the medium pressure steam of the medium pressure section to a low pressure steam for delivery to the low pressure section, wherein the low pressure steam has a pressure of greater than or equal to 4 bar and less than 25 bar and a temperature of greater than or equal to 140 °C and less than 220 °C; and, wherein the central heat recovery system delivers low pressure steam to the heat exchange system of the methanol distillation column and the drying unit.

Clause 21. The system of any one of clauses 17 to 20, further comprising at least one of:

an oxygen compressor upstream of the gasification reactor for increasing the pressure of the oxygen feed,
a syngas compressor upstream of the methanol synthesis reactor for increasing the pressure of the hydrogen-rich syngas,
a hydrogen compressor upstream of the methanol synthesis reactor for increasing the pressure of a hydrogen feed prior to the hydrogen feed entering the methanol synthesis reactor, and
a carbon dioxide compressor for pressurising carbon dioxide discharged from the acid-gas removal unit;

wherein at least one of the oxygen compressor, syngas compressor, hydrogen compressor and carbon dioxide compressor comprises a heat exchange system for recovering hot water and/or condensate and providing the hot water and/or condensate to a hot water and/or condensate section of the central heat recovery system,
optionally, wherein the central heat recovery system delivers hot water and/or condensate from the hot water and/or condensate section back to the heat exchange system of at least one of the oxygen compressor, syngas compressor, hydrogen compressor and carbon dioxide compressor.

Clause 22. The system of clause 21, wherein the hot water and/or condensate section of the central heat recovery system receives hot water and/or condensate from at least one of the heat exchange system of the acid gas removal unit, methanol distillation column and the drying unit.

Clause 23. The system of any one of clauses 17 to 22, further comprising at least one steam turbine for expanding steam supplied by the central heat recovery system to produce shaft power for mechanical work.

Clause 24. The system of clause 23 when dependent on clause 21 or clause 22, wherein the at least one steam turbine produces shaft power to drive at least one of the oxygen compressor, syngas compressor, hydrogen compressor and carbon dioxide compressor.

Clause 25. A method of recovering heat from the system of any one of clauses 20 to 26, the method comprising:

maintaining the temperature of the gasification reactor and first methanol synthesis reactor using the heat exchange system of the gasification reactor and/or methanol synthesis reactor and producing steam using the recovered heat;
delivering the steam to the central heat recovery system;
directing steam from the central heat recovery system to the heat exchange system of each of the drying unit, acid-gas removal unit and methanol distillation column to heat the drying unit, acid-gas removal unit and methanol distillation column.

Clause 26. A method of converting biomass to methanol in a system comprising:

a drying unit, a torrefaction unit, a gasification reactor and a methanol synthesis reactor,
wherein the method comprises:

drying a raw biomass feed in the drying unit to produce a dried biomass feed;
converting the dried biomass feed to a torrefied biomass feed in a torrefaction process in the torrefaction unit;
converting the torrefied biomass feed to a syngas feed in a gasification process in the gasification reactor; and,
converting at least a portion of the syngas feed to methanol in the methanol synthesis reactor;

wherein slag is produced as a waste product of the gasification process, and the method comprises recycling at least a portion of the slag with at least one of the raw biomass feed, the dried biomass feed, and the torrefied biomass feed upstream of the gasification reactor.

Clause 27. The method of clause 26, wherein the first portion of the slag is combined with at least one of the raw biomass feed, the dried biomass feed, and the torrefied biomass feed upstream of the gasification reactor at a rate of 2.5 to 4.0 tonnes of slag per hour, preferably 3.0 to 3.5 tonnes of slag per hour.

Clause 28. The method of clause 26 or clause 27, wherein the first portion of the slag is combined with the torrefied biomass downstream of the torrefaction unit and upstream of the gasification reactor.

Clause 29. The method of any one of clauses 26 to 28, wherein a second portion of the slag is removed from the system downstream of the gasification reactor.

Clause 30. The method of any one or clauses 26 to 29, wherein the torrefaction process produces a volatiles stream and the method further comprises:

directing at least a portion of the volatiles stream to a combustor and combusting the portion of the volatiles stream in the combustor to produce a hot exhaust stream;
transferring heat from the hot exhaust stream to an air stream in a heat exchange system to produce a hot air stream; and,
supplying at least a portion of the hot air stream to the drying unit to provide heat for drying the raw biomass feed.

Clause 31. The method of clause 30, comprising supplying at least a further portion of the volatiles stream to the torrefaction unit to be used as a fuel for providing heat for the torrefaction process.

Clause 32. The method of any one of clauses 26 to 31, comprising recovering heat from water vapour released in the drying of the raw biomass feed using a condenser and using the recovered heat to provide heat to the drying process.

Clause 33. A system for converting biomass to methanol in a process according to any one of clauses 26 to 32, the system comprising:

a drying unit for drying a raw biomass feed to produce a dried biomass effluent;
a torrefaction unit for converting the dried biomass effluent to a torrefied biomass effluent in a torrefaction process;
a gasification reactor for converting the torrefied biomass effluent to a syngas stream, in a gasification process;

and,

a methanol synthesis reactor for converting the syngas stream to methanol;

wherein the gasification reactor comprises an outlet for discharging slag produced in the gasification process, and the system further comprises a conduit for directing a portion of the slag from the outlet of the gasification reactor to a position upstream of the gasification reactor for mixing with at least one of the raw biomass feed, the dried biomass effluent, and the torrefied biomass effluent.

Clause 34. The system of clause 33, wherein the torrefaction unit comprises a volatiles stream outlet for discharging a volatiles stream produced in the torrefaction process, and the system further comprises:

a combustor for combusting at least a portion of the volatiles stream to produce a hot exhaust stream; and,
a heat exchange system for transferring heat from the hot exhaust stream to an air stream to produce a hot air stream;
wherein the drying unit is configured to receive the hot air stream to provide heat for drying the raw biomass feed.

Clause 35. The system of any one of clauses 33 or 34, further comprising a heat recovery system comprising a condenser for recovering heat from water vapour released in the drying of the raw biomass feed, and being configured to provide recovered heat to the drying unit.

## Example

The maximum methanol yield produced in a system according to Figure 1 was estimated for the system operating in gasification-only mode, full-carbon recovery mode and an intermediate mode. The maximum input/output capacity of various components of the system were obtained from technology providers performance data, literature or otherwise simulated by process software such as Aspen Plus.

Table 2 shows the maximum input or output capacity of various stages and components of a system according to Figure 1. At a maximum input of 308 t/h (tonnes per hour) raw wet biomass into the system, it is estimated that 116 t/h of processed biomass (feed dust) is produced in the biomass processing stages upstream of the gasification reactor and provided as an input to the gasifier. The maximum syngas input to the water-gas shift reactor when the water-gas shift reactor is an entrained flow gasifier, is 325 t/h and the maximum output of syngas from the water-gas shift reactor is 231 t/h. The maximum output from the acid-gas removal unit is estimated to be 82 t/h.

When the system is operated in gasification-only mode, the maximum crude methanol output from the methanol synthesis reactor is estimated to be 75 t/h, leading to a recovery of 70 t/h of distillate methanol from the distillation column.

When the system is operated in full-carbon recovery mode, the maximum crude methanol output from the methanol synthesis reactor is estimated to be 175 t/h, leading to a recovery of 165 t/h of distillate methanol from the distillation column. The significantly higher (approximately 100 t/h) methanol yield in full-carbon recovery mode is at least in part due to the bypass of the water-gas shift reactor and thus minimised loss of carbon content of the syngas as biogenic carbon dioxide.

**Table 2: Maximum input/output capacity**

| Stage | Maximum input/output |
|---|---|
| Raw wet biomass input | 308 t/h |
| Biomass (feed dust) input to gasifier | 116 t/h @ 85 °C |
| Syngas input to water-gas shift reactor | 325 t/h @ 210 °C, 38 bar |
| Syngas output from water-gas shift reactor | 231 t/h @ 40 °C, 33 bar |
| Syngas output from acid-gas removal unit | 82 t/h @ 50 °C, 30 bar |
| Crude methanol (Gasification only mode) | 75 t/h @ 79 °C, 6 bar |
| Distillate methanol (Gasification only mode) | 70 t/h @ 41 °C, 1 bar |
| Crude methanol (Full-carbon recovery mode) | 175 t/h @ 75 °C, 6 bar |
| Distillate methanol (Full-carbon recovery mode) | 165 t/h @ 41 °C, 1 bar |
| Hydrogen (from electrolysis unit) consumed (Full carbon recovery mode) | 18.3 t/h |

It is desirable to operate the system in full-carbon recovery mode for as much time as possible in order to maximise methanol yield. However, full-recovery mode depends on operation of the electrolysis unit, powered by green electricity. Green electricity supply and price is prone to fluctuate. The energy demands of the electrolysis unit is considerable and is estimated to be 962 MW in full carbon capture mode produces 18.3 t/h of hydrogen. In full-carbon recovery mode, the electrolysis unit achieves maximum methanol production and a surplus of oxygen. Therefore, a fluctuation in supply and price of green electricity can significantly impact the economics of full-carbon recovery mode. In contrast, the energy requirements of the air-separation unit is estimated to be 25 MW to 45 MW to meet the oxygen demand of the gasification reactor. Therefore, when the price of green electricity is such that full-carbon recover mode is not economically viable, the system may be operated in gasification only mode or intermediate mode as described herein.

The system may be operated in gasification only, intermediate or full carbon recovery mode as described herein and illustrated in Table 3. In each mode, the oxygen demand of the biomass consumption of the system and therefore oxygen demand of the gasifier, remains constant.

In gasification only mode, the electrolysis unit is not operated and all syngas is passed through the water-gas shift reactor to increase the hydrogen content of the syngas. Carbon dioxide produced in the water-gas shift process is removed by the acid-gas removal unit as biogenic carbon dioxide. The carbon content lost as biogenic carbon dioxide limits the methanol (distillate) yield of the process to 70 t/h.

In full carbon recovery mode, the electrolysis unit is operational and produces 18.3 t/h of hydrogen. The hydrogen produced meets the stoichiometric demand of the system so that all syngas is passed through the bypass conduit and does not pass through the water-gas shift unit. As a consequence, carbon content is not lost as biogenic carbon and the methanol distillate yield of the process is 165 t/h.

The system may be operated in an intermediate mode between gasification mode and full carbon mode. It will be understood that the intermediate mode is not a discrete mode but a continuum between the extremes of gasification only and full carbon recovery mode. In intermediate mode, the electrolysis unit produces >0 t/h of hydrogen to < 18.3 t/h of hydrogen. The biogenic carbon removed by the acid-gas removal unit therefore varies from >0 t/h to <121 t/h and the methanol (distillate) yield also varies from >70 to < 165 t/h. In an intermediate mode, the oxygen required for gasification reactor may be supplied by a combination of the electrolysis unit and the air-handling unit. The power requirement of the electrolysis unit to replace the air-handling unit is 426 MW.

Table 3: Comparison of gasification only, intermediate and full carbon recover modes.

|  | Gasification Only Mode | Intermediate Mode | Full Carbon Recovery Mode |
|---|---|---|---|
| Biomass consumption (as received) | 285 - 310 t/h | 285 - 310 t/h | 285 - 310 t/h |
| $O_2$ demand of gasifier | 60 - 65 t/h | 60-65 t/h | 60-65 t/h |
| $H_2$ produced by electrolysis unit | 0 t/h | 0 →18.3 t/h | 18.3 t/h |
| Biogenic $CO_2$ surplus (removed by AGR) | 121 t/h | 121→0 t/h | 0 t/h |
| Methanol (distillate) Production | 70 t/h | 70→165 t/h | 165 t/h |

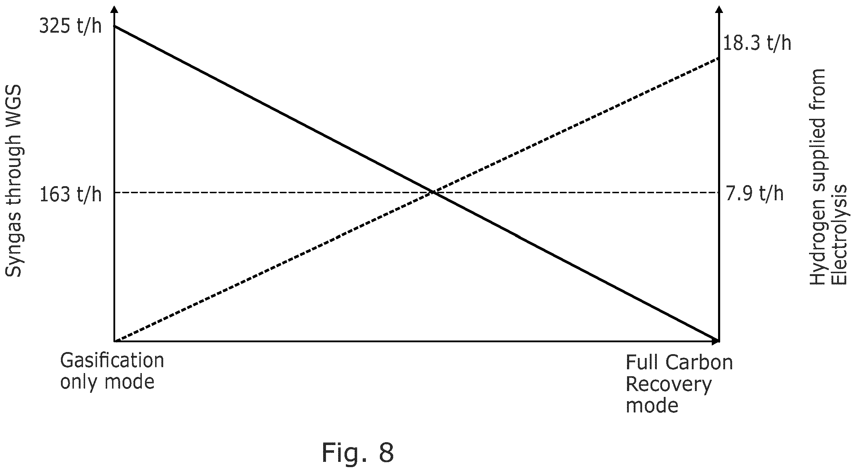

The bypass control system adjusts the flow of syngas through the bypass conduit and the flow of syngas to the water-gas shift reactor. depending on the operational mode of the system. Figure 8 shows the change in hydrogen flow (diagonal dashed line in Fig. 8) and syngas flow through the water-gas shift reactor (diagonal solid line in Figure 8) in each operational mode. As illustrated in Figure 8, when the electrolysis unit is not operated (hydrogen supplied is 0t/h), then the system is operated in gasification-only mode and the control valve is positioned so that all syngas (325 t/h) is passed through the water-gas shift reactor and no syngas is passed through the bypass conduit. In full carbon recovery mode, the electrolysis unit is operated to produce 18.3 t/h of hydrogen, meeting the stoichiometric demand of the system. The bypass valve is position so that no syngas is passed through the water-gas shift reactor and all syngas is passed through the bypass conduit. The intermediate mode is the continuum between the gasification only and full carbon recovery modes at the extremes.

As the hydrogen output of the electrolysis unit is varied (e.g. in response of green electricity supply and price), the bypass valve is adjusted to vary the amount of syngas through the water-gas shift reactor to maintain stoichiometric ratio. When the electrolysis unit is operated so that 7.9 t/h of hydrogen is produced, the bypass valve is positioned so that approximately 50% of the syngas is passed through the water-gas shift reactor and approximately 50% of the syngas is passed through the bypass conduit.

**Claims**

1. A system for converting biomass to methanol, the system comprising:

a gasification reactor comprising a biomass feed inlet and a syngas outlet;
a water-gas shift reactor located downstream of the gasification reactor and comprising a syngas stream inlet and hydrogen-rich syngas stream outlet;
a first methanol synthesis reactor located downstream of the water-gas shift reactor;
a bypass conduit fluidly connecting the syngas outlet of the gasification reactor with the first methanol synthesis reactor, wherein the bypass conduit does not pass through the water-gas shift reactor;
a bypass control system for controlling the flow of syngas to the water-gas shift reactor and the flow of syngas through the bypass conduit;
an air-separation unit comprising an air inlet and an oxygen outlet; and,
an electrolysis unit comprising a water inlet, an oxygen outlet and a hydrogen outlet;

wherein the gasification reactor is arranged to receive:

(i) oxygen from the oxygen outlet of the air-separation unit; and/or,
(ii) oxygen from the oxygen outlet of the electrolysis unit;
and the first methanol synthesis reactor is arranged to receive:
(iii) hydrogen-rich syngas from the hydrogen-rich syngas stream outlet of water-gas shift reactor; and/or,
(iv) syngas from the bypass conduit and hydrogen from the hydrogen outlet of the electrolysis unit.

2. The system of claim 1, wherein the gasification reactor is an entrained flow gasifier, optionally wherein the entrained flow gasifier has a maximum syngas stream output of at least 300 t/h; or,
wherein the gasification reactor is a fluidized bed gasifier, optionally wherein the fluidized bed gasifier has a maximum syngas stream output of at least 80 t/h.

3. The system of any preceding claim, further comprising a first distillation column for producing a methanol distillate, wherein the first distillation column comprises an inlet for receiving methanol effluent from the first methanol synthesis reactor and an outlet for discharging a methanol distillate having a higher content of methanol by weight % than the methanol effluent;
optionally, wherein the methanol distillate has a methanol content of at least 99.85 wt% methanol effluent.

4. The system of any preceding claim, further comprising a second and a third methanol synthesis reactor for converting syngas to methanol;

wherein the first methanol synthesis reactor and the second methanol synthesis reactor are arranged in parallel so that both of the first and second methanol synthesis reactors are arranged to receive:

(iii) the hydrogen-rich syngas from the hydrogen-rich syngas stream outlet of water-gas shift reactor; and/or,
(iv) the syngas from the bypass conduit and hydrogen from the

hydrogen outlet of the electrolysis unit; and,

wherein each of the first and second methanol synthesis reactors comprise:

an outlet for discharging methanol; and,
an outlet for discharging unreacted syngas;
wherein the third methanol synthesis reactor is arranged in series with the first and second methanol synthesis reactor, and is arranged to receive methanol effluent from the outlet of the first methanol synthesis reactor and/or the outlet of the second methanol synthesis reactor; and, an outlet for discharging a methanol effluent,
optionally, wherein the system further comprises a second and third methanol distillation column for producing a methanol distillate;
wherein the inlet of the first methanol distillation column is for receiving methanol effluent from the first methanol synthesis reactor;
the second methanol distillation column has an inlet for receiving methanol effluent from the second methanol synthesis reactor; and,

the third methanol distillation column has an inlet for receiving methanol effluent from the third methanol synthesis reactor;

wherein each of the first, second and third methanol distillation columns comprise an outlet for discharging a methanol distillate having a higher content of methanol by weight % than the methanol effluent;

optionally, wherein the methanol distillate of each methanol distillation column has a methanol content of at least 99.85 wt% methanol effluent.

5. The system according to any preceding claim, further comprising an acid gas removal unit for removing acid gases from syngas, wherein the acid gas removal unit is located downstream of the water-gas shift reactor and upstream of the first methanol synthesis reactor,

wherein the acid gas removal unit has an inlet for receiving hydrogen-rich syngas from the water-gas shift reactor, and optionally syngas from the bypass conduit; an absorber/desorber system for acid gas recovery; an outlet for discharging recovered acid gas; and an outlet for cleaned syngas;

optionally, wherein the recovered acid gas comprises carbon dioxide and the absorber/desorber system comprises an amine-absorber/desorber system.

6. A method for converting biomass to methanol in a system comprising:

a gasification reactor;

a water-gas shift reactor located downstream of the gasification reactor;

a first methanol synthesis reactor located downstream of the water-gas shift reactor;

a bypass conduit for diverting syngas from upstream of the water-gas shift reactor to downstream of the water-gas shift reactor and without passing through the water-gas shift reactor;

a bypass control system for controlling the flow of syngas to the water-gas shift reactor and the flow of syngas through the bypass conduit;

an air-separation unit for supplying oxygen to the gasification reactor; and,

an electrolysis unit for supplying oxygen to the gasification reactor and hydrogen to a first methanol synthesis reactor;

wherein the method comprises: converting a biomass feed and an oxygen feed to a syngas stream in the gasification reactor;

feeding at least a portion of the syngas stream to the water-gas shift reactor to produce a hydrogen-rich syngas stream;

operating the electrolysis unit to produce a hydrogen stream;

feeding at least a portion of the syngas stream through the bypass conduit and without passing through the water-gas shift reactor;

combining the hydrogen-rich syngas stream, syngas from the bypass conduit and the hydrogen stream to form a combined stream; and,

converting the combined stream to methanol in the first methanol synthesis reactor;

wherein the method further comprises:

adjusting the hydrogen stream output of the electrolysis unit; and,

operating the bypass control system to maintain the stoichiometric number of the combined stream within a predetermined range, wherein the stoichiometric number (SN) is defined as: $SN = ([H_2] - [CO_2])/([CO] + [CO_2])$

and $[H_2]$ is the hydrogen concentration of the combined stream, $[CO_2]$ is the carbon dioxide concentration of the combined stream and $[CO]$ is the carbon monoxide concentration of the combined stream.

7. The method of claim 6, wherein the predetermined range is 2.0 to 2.1.

8. The method of claim 6 or claim 7, further comprising:

operating the bypass control system so that all of the syngas stream passes through the water-gas shift reactor to form the hydrogen-rich syngas, and converting the hydrogen-rich syngas stream to methanol in the first methanol synthesis reactor; or,

operating the bypass control system so that all of the syngas stream passes through the bypass conduit, and converting the syngas from the bypass conduit and the hydrogen stream from the electrolysis unit to methanol in the first methanol synthesis reactor.

9. The method of any preceding claim, wherein the air-separation unit supplies at least a portion of the oxygen feed of the gasification reactor and the electrolysis unit also supplies at least a portion of the oxygen feed of the gasification reactor, and the method further comprises:
adjusting the flow of oxygen from the air-separation unit to the gasification reactor and the flow of oxygen from the electrolysis unit to the gasification reactor in response to the adjustment of the hydrogen stream output of the electrolysis unit.

10. The method of claim 9, wherein when the hydrogen stream output of the electrolysis unit is increased, the method further comprises increasing the flow of oxygen from the electrolysis unit to the gasification reactor and decreasing the flow of oxygen from the air-separation unit to the gasification reactor; or,
when the hydrogen stream output of the electrolysis unit is decreased, the method further comprises decreasing the flow of oxygen from the electrolysis unit to the gasification reactor and increasing the flow of oxygen from the air-separation unit to the gasification reactor.

11. The method of any one of claims 6 to 10, wherein the system comprises a sample outlet for venting a sample of the combined stream and a detector for determining the stoichiometric number of the sample of the combined stream; and,
the bypass control system further comprises a controller and at least one control valve and operating the bypass control system to maintain the stoichiometric number of the combined stream within a predetermined range comprises:

determining the stoichiometric number of a sample of the combined stream vented from the sample outlet using the detector;
providing the stoichiometric number of the combined stream as a data input into the controller;
comparing the stoichiometric number of the combined syngas to the predetermined range stored on the controller as a reference stoichiometric range and,
when the stoichiometric number of the combined stream is outside of the reference stoichiometric range, adjusting the at least one control valve to adjust the flow of the syngas to the water-gas shift reactor and/or the flow of syngas through the bypass conduit.

12. A system for converting biomass to methanol, the system comprising:

a drying unit for drying raw biomass to produce a dried biomass feed;
a gasification reactor for converting the dried biomass feed and an oxygen feed to syngas;
a water-gas shift reactor for increasing the hydrogen content of the syngas to form a hydrogen-rich syngas stream;
an acid gas removal unit for removing acidic gases from the hydrogen-rich syngas stream;
a first methanol synthesis reactor for converting the hydrogen-rich syngas to crude methanol; and,
a methanol distillation column for purifying the crude methanol to produce a methanol distillate;
wherein each of the gasification reactor and first methanol synthesis reactor comprise a heat exchange system for controlling the temperature of the gasification reactor and/or methanol synthesis reactor and producing steam using recovered heat; and,
each of the drying unit, acid-gas removal unit and methanol distillation column comprise a heat exchange system for transferring heat from the steam to the drying unit, acid-gas removal unit or methanol distillation column;
wherein the system further comprises a central heat recovery system for receiving the steam from the heat exchange systems of the gasification reactor and first methanol synthesis reactor and delivering the steam to the heat exchange system of each of the drying unit, acid-gas removal unit and methanol distillation column.

13. The system of claim 12, wherein the heat exchange system of the gasification reactor produces a high pressure steam having a pressure of at least 45 bar and a temperature of at least 255 °C; and,

the central heat recovery system comprises a high pressure steam section for receiving the high pressure steam and delivering the high pressure steam to the heat exchange system of the acid-gas removal unit; and,
wherein the heat exchange system of the first methanol synthesis reactor produces a medium pressure steam having a pressure of greater than or equal to 25 bar and less than 45 bar and a temperature of greater than or equal to 220 °C and less than 255 °C; and,
the central heat recovery system comprises a medium pressure steam section for receiving the medium pressure steam and delivering the medium pressure steam to the heat exchange system of the methanol distillation column.

14. The system of claim 13, wherein the central heat recovery system comprises a cascading stage between the high pressure steam section and medium pressure steam section for expanding a portion of the high pressure steam of the high pressure section to a medium pressure steam;

optionally, wherein the central heat recovery system comprises a cascading stage between the medium pressure steam section and a low pressure steam section for expanding a portion of the medium pressure steam of the medium pressure section to a low pressure steam for delivery to the low pressure section, wherein the low pressure steam has a pressure of greater than or equal to 4 bar and less than 25 bar and a temperature of greater than or equal to 140 °C and less than 220 °C; and, wherein the central heat recovery system delivers low pressure steam to the heat exchange system of the methanol distillation column and the drying unit.

15. The system of any one of claims 12 to 14, further comprising at least one of:

an oxygen compressor upstream of the gasification reactor for increasing the pressure of the oxygen feed,
a syngas compressor upstream of the methanol synthesis reactor for increasing the pressure of the hydrogen-rich syngas,
a hydrogen compressor upstream of the methanol synthesis reactor for increasing the pressure of a hydrogen feed prior to the hydrogen feed entering the methanol synthesis reactor, and
a carbon dioxide compressor for pressurising carbon dioxide discharged from the acid-gas removal unit;

wherein at least one of the oxygen compressor, syngas compressor, hydrogen compressor and carbon dioxide compressor comprises a heat exchange system for recovering hot water and/or condensate and providing the hot water and/or condensate to a hot water and/or condensate section of the central heat recovery system,

optionally, wherein the central heat recovery system delivers hot water and/or condensate from the hot water and/or condensate section back to the heat exchange system of at least one of the oxygen compressor, syngas compressor, hydrogen compressor and carbon dioxide compressor.

16. A method of recovering heat from the system of any one of claims 14 to 24, the method comprising:

maintaining the temperature of the gasification reactor and first methanol synthesis reactor using the heat exchange system of the gasification reactor and/or methanol synthesis reactor and producing steam using the recovered heat;
delivering the steam to the central heat recovery system;
directing steam from the central heat recovery system to the heat exchange system of each of the drying unit, acid-gas removal unit and methanol distillation column to heat the drying unit, acid-gas removal unit and methanol distillation column.

17. A method of converting biomass to methanol in a system comprising:

a drying unit, a torrefaction unit, a gasification reactor and a methanol synthesis reactor,
wherein the method comprises:

drying a raw biomass feed in the drying unit to produce a dried biomass feed;
converting the dried biomass feed to a torrefied biomass feed in a torrefaction process in the torrefaction unit;
converting the torrefied biomass feed to a syngas feed in a gasification process in the gasification reactor; and,
converting at least a portion of the syngas feed to methanol in the methanol synthesis reactor;

wherein slag is produced as a waste product of the gasification process, and the method comprises recycling at least a portion of the slag with at least one of the raw biomass feed, the dried biomass feed, and the torrefied biomass feed upstream of the gasification reactor.

18. The method of claim 17, wherein the first portion of the slag is combined with at least one of the raw biomass feed, the dried biomass feed, and the torrefied biomass feed upstream of the gasification reactor at a rate of 2.5 to 4.0 tonnes of slag per hour, preferably 3.0 to 3.5 tonnes of slag per hour.

19. The method of any one or claims 17 or 18, wherein the torrefaction process produces a volatiles stream and the method further comprises:

directing at least a portion of the volatiles stream to a combustor and combusting the portion of the volatiles stream in the combustor to produce a hot exhaust stream;
transferring heat from the hot exhaust stream to an air stream in a heat exchange system to produce a hot air stream; and,
supplying at least a portion of the hot air stream to the drying unit to provide heat for drying the raw biomass feed.

20. The method of claim 19, comprising supplying at least a further portion of the volatiles stream to the torrefaction unit to be used as a fuel for providing heat for the torrefaction process.

21. A system for converting biomass to methanol in a process according to any one of claims 17 to 20, the system comprising:

a drying unit for drying a raw biomass feed to produce a dried biomass effluent;
a torrefaction unit for converting the dried biomass effluent to a torrefied biomass effluent in a torrefaction process;
a gasification reactor for converting the torrefied biomass effluent to a syngas stream, in a gasification process; and,
a methanol synthesis reactor for converting the syngas stream to methanol;
wherein the gasification reactor comprises an outlet for discharging slag produced in the gasification process, and the system further comprises a conduit for directing a portion of the slag from the outlet of the gasification reactor to a position upstream of the gasification reactor for mixing with at least one of the raw biomass feed, the dried biomass effluent, and the torrefied biomass effluent.

22. The system of claim 21, wherein the torrefaction unit comprises a volatiles stream outlet for discharging a volatiles stream produced in the torrefaction process, and the system further comprises:

a combustor for combusting at least a portion of the volatiles stream to produce a hot exhaust stream; and,
a heat exchange system for transferring heat from the hot exhaust stream to an air stream to produce a hot air stream;
wherein the drying unit is configured to receive the hot air stream to provide heat for drying the raw biomass feed.

Fig. 1

Fig. 2

Fig. 3

Fig. 4

Fig. 5

EP 4 763 828 A1

600

601 - gasification process

603 - water-gas shift process

605 - operated electrolysis unit

607 - bypassing syngas

609 - combining streams

611 - methanol synthesis

613 - adjusting hydrogen output of electrolysis unit

615 - operate bypass control system

Fig. 6

700

```
┌─────────────────────────────────────────┐
│        701 - drying biomass              │
└─────────────────────────────────────────┘
                   │
                   ▼
┌─────────────────────────────────────────┐
│        703 - torrefaction process        │◄──┐
└─────────────────────────────────────────┘   │
                   │                           │
                   ▼                           │
┌─────────────────────────────────────────┐   │
│        705 - gasification process         │   │
└─────────────────────────────────────────┘   │
                   │                           │
                   ▼                           │
┌─────────────────────────────────────────┐   │
│        707 - methanol synthesis          │   │
└─────────────────────────────────────────┘   │
                   │                           │
                   ▼                           │
┌─────────────────────────────────────────┐   │
│        709 - slag recycle                │───┘
└─────────────────────────────────────────┘
```

Fig. 7

Fig. 8

Europäisches
Patentamt
European
Patent Office
Office européen
des brevets

# EUROPEAN SEARCH REPORT

**Application Number**

EP 25 15 5326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2023/217703 A1 (TOPSOE AS [DK]) 16 November 2023 (2023-11-16) * page 3, lines 11-23 * * page 6, lines 11-28 * * page 11, lines 20-23, 28, 30 * * page 12, line 5 * * figure 2 * * page 5, lines 19-29 * ----- | 1-11 | INV. C07C29/151 C07C31/04 |
| Y | US 2013/143972 A1 (TOWNSEND DAVID W [US] ET AL) 6 June 2013 (2013-06-06) * paragraphs [0003], [0021] - [0022], [0043], [0091] * * figure 4 * ----- | 1-11 | |

**TECHNICAL FIELDS SEARCHED    (IPC)**

C07C

The present search report has been drawn up for all claims

| Place of search | Date of completion of the search | Examiner |
|---|---|---|
| Munich | 2 July 2025 | Fitz, Wolfgang |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

&: member of the same patent family, corresponding
   document

EPO FORM 1503 03.82 (P04C01)

Europäisches
Patentamt

European
Patent Office

Office européen
des brevets

**Application Number**

EP 25 15 5326

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-11

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

The Search Division considers that the present European patent application does not comply with the requirements of unity of invention and relates to several inventions or groups of inventions, namely:

      1. claims: 1-11

          System and method for converting biomass to methanol involving a gasification reactor, a water-gas shift reactor, a methanol synthesis reactor, a bypass conduit system which does not pass through the water-gas shift reactor, an air separation unit and an electrolysis unit

          ---

      2. claims: 12-16

          System for converting biomass to methanol involving a drying unit, a gasification reactor, a water-gas shift reactor, an acid gas removal unit, a methanol synthesis reactor, a methanol distillation column with a heat exchange set-up (comprising the gasification reactor, methanol synthesis reactor, drying unit, acid-gas removal unit and methanol distillation column); a method of recovering heat from the system

          ---

      3. claims: 17-22

          Method of and system for converting biomass to methanol involving a drying unit, a torrefaction unit, a gasification reactor and a methanol synthesis reactor wherein slag produced as a waste product of the gasification is recycled as specified in claim 17

          ---

## ANNEX TO THE EUROPEAN SEARCH REPORT
## ON EUROPEAN PATENT APPLICATION NO.

EP 25 15 5326

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2025

| Patent document cited in search report | Publication date | Patent family member(s) | | Publication date |
|---|---|---|---|---|
| WO 2023217703 A1 | 16-11-2023 | AR | 129254 A1 | 07-08-2024 |
| | | AU | 2023266683 A1 | 12-12-2024 |
| | | CL | 2024003425 A1 | 04-04-2025 |
| | | CN | 119183437 A | 24-12-2024 |
| | | EP | 4522557 A1 | 19-03-2025 |
| | | KR | 20250010614 A | 21-01-2025 |
| | | TW | 202348548 A | 16-12-2023 |
| | | WO | 2023217703 A1 | 16-11-2023 |
| US 2013143972 A1 | 06-06-2013 | US | 2013143972 A1 | 06-06-2013 |
| | | US | 2013143973 A1 | 06-06-2013 |
| | | US | 2013144087 A1 | 06-06-2013 |

EPO FORM P0459

For more details about this annex : see Official Journal of the European Patent Office, No. 12/82

**REFERENCES CITED IN THE DESCRIPTION**

**Patent documents cited in the description**

- US 20220220052 A1
- US 9416077 B2